# NOUVEAU FASCICULE DE BREVET EUROPEEN

(11) **EP 0 861 266 B3**
(45) Date de publication du présent fascicule: **08.04.2009**
(45) Mention de la délivrance du brevet: 23.04.2003
(21) Numéro de dépôt: 96939132.5
(22) Date de dépôt: 15.11.1996
(51) Int. Cl.: C07K 14/66, C07K 7/06, A61K 38/04

(54) **CONJUGUES PEPTIDIQUES DERIVES DES HORMONES THYMIQUES, LEUR UTILISATION A TITRE DE MEDICAMENT ET COMPOSITIONS LES CONTENANT**
AUS THYMISCHEN HORMONEN ABSTAMMENDE PEPTID KONJUGIERTE, DEREN VERWENDUNG ALS MEDIKAMENTE UND AUS DEREN ENTHALTENDE ZUSAMMENSETZUNGEN
PEPTIDE CONJUGATES DERIVED FROM THYMIC HORMONES, THEIR USE AS MEDICAMENT AND COMPOSITIONS CONTAINING THEM

(30) Priorité: 15.11.1995 FR 9513544
(43) Date de publication de la demande: 02.09.1998
(73) Titulaire: INSTITUT EUROPEEN DE BIOLOGIE CELLULAIRE, 31520 Ramonville Saint-Agne (FR)
(72) Inventeur: DUSSOURD D'HINTERLAND, Lucien, F-31400 Toulouse (FR); PINEL, Anne-Marie, F-34280 La Grande-Motte (FR)
(74) Mandataire: Warcoin, Jacques
(86) Numéro de dépôt international: PCT/FR1996/001812
(87) Numéro de publication internationale: WO 1997/018239

(56) Documents cités:
- EP-A- 0 166 612
- FR-A- 2 411 174
- CHEMICAL ABSTRACTS, vol. 122, no. 1, 2 Janvier 1995 Columbus, Ohio, US; abstract no. 1237g, J ODARJUK ET AL.: "Histamine-releasing activity as an undesired side-effect in the development of peptide analogs" page 147; XP002010397 & CHEM. PEPT. PROTEINS, vol. 5/6 (Pt. A), 1993, pages 475-482,
- REGULATORY PEPTIDES, vol. 27, 1990, AMSTERDAM, THE NETHERLANDS, pages 257-262, XP000646225 G A HEAVNER ET AL.: "Peptide analogs of thymopentin distinguish distinct thymopoietin receptor specificities on two human T cell lines"
- PEPTIDES, vol. 7, no. 6, Décembre 1986, FAYETTEVILLE, USA, pages 1015-1019, XP000646219 G A HEAVNER ET AL.: "Biologically active analogs of thymopentin with enhanced enzymatic stability"
- CHEMICAL ABSTRACTS, vol. 104, no. 3, 20 Janvier 1986 Columbus, Ohio, US; abstract no. 19826, "Peptides for radioimmunoassay of serum thymic factor" XP002027989 & JP 60 089 499 A (MITSUI PHARMACEUTICALS) 20 Mai 1985

## Description

L'invention se rapporte à de nouveaux dérivés synthétiques de deux hormones thymiques, la thymuline et la thymosine, et à leurs applications, en tant que médicament ou dans le domaine de la cosmétologie.

Les nombreuses études sur le fonctionnement du système immunitaire, ont mis en évidence le rôle capital du thymus et des hormones thymiques, dans la stimulation des défenses immunitaires de l'organisme.

Les hormones thymiques, en particulier la thymuline, "J.F. BACH et coll.", la thymopoïétine "GRANDSTEIN" et la Thymosine "DAYNE" ont été identifiées comme étant de nature polypeptidique et peptidique.

Ces hormones et principalement la thymuline et la thymopoïétine, ont la propriété d'induire la maturation des lymphocytes T et de stimuler la réponse immunitaire de l'organisme.

Il a été démontré scientifiquement qu'il existait une relation étroite entre la diminution des fonctions thymiques avec l'âge, et le vieillissement cutané sur le plan physiologique normal.

Au niveau pathologique, la perte d'activité du thymus, "involution", se traduit par l'apparition de troubles physiologiques : "maladies auto-immunes, alopécies (chute anormale des cheveux) etc...".

Il a également été démontré qu'il existait une relation entre les propriétés physiologiques du thymus et certaines cellules de l'épiderme.

Il existe en effet une similitude de structure entre certaines cellules du thymus et les kératinocytes. Il a été démontré que les kératinocytes sécrètent également, dans certaines conditions, une hormone du type thymopoietine identique à celle sécrétée par le thymus, et sont capables d'induire une maturation lymphocytaire post thymique, et de prendre ainsi le relais du thymus.

Cette relation, entre les activités physiologiques des cellule du thymus et les kératinocytes, a été récemment confirmée par la mise en évidence dans les cellules du thymus, de la présence de kératine et de kératohyaline, de structures identiques à la kératine et à la kératohyaline sécrétée par les kératinocytes de l'épiderme.

Par ailleurs la démonstration apportée sur le plan scientifique, que les kératinocytes en phase de différenciation sécrétaient une substance activant la croissance des thymocytes, l'ETAF confirme les relations physiologiques existant entre le thymus et les kératinocytes. (ETAF : Epidermal Thymocyte Activating Factor).

Il existe un parallèle entre "l'involution" du thymus et la diminution de la synthèse de l'ETAF par les kératinocytes en fonction de l'âge, avec toutefois un décalage fonctionnel en faveur des kératinocytes (maturation lymphocytaire post thymique).

L'utilisation des hormones thymiques, en particulier la thymuline et la Thymopoïetine en thérapeutique, a fait l'objet de nombreux travaux pharmacologiques et cliniques, en vue de la stimulation et de la régulation des défenses immunitaires de l'organisme.

Utilisées sous forme d'extraits ou de molécules obtenues par synthèse chimique, ces produits ont donnés des résultats irréguliers et décevants, par suite de leur manque de stabilité, et d'un effet ubiquitaire lié à l'absence de relations doses-effets.

La demande de brevet FR 2 411 174 divulgue une séquence d'aminoacide - ALA-LYS-SER-GLN- qui présente une activité biologique par injection, utile dans le système immune des être humains et des animaux.

La demande de brevet EP 166 612 décrit des penta-peptides dérivés de la thymopoïétine, utiles dans le traitement des maladies impliquant des infections chroniques.

La présente invention a pour objet, l'obtention par synthèse chimique de dérivés peptidiques des hormones thymiques, dont la structure a été orientée de façon à leur assurer une grande stabilité physico-chimique, et une activité pharmacologique parfaitement définie.

Utilisant l'ensemble des connaissances acquises et développées récemment par nos équipes, concernant le rôle primordial de l'épiderme dans les défenses immunitaires et ses relations avec le thymus (International Society of Development and Comparative Immunology), la présente invention a pour objet, la réalisation de dérivés peptidiques de synthèse, dont l'activité physiologique etthérapeutique est orientée vers la stimulation et la modulation des défenses immunitaires du derme et de l'épiderme préférentiellement à toute action systémique.

Un des objets péférentiel de l'invention concerne la réalisation par synthèse chimique de métallopeptides dont les propriétés physiologiques sont spécifiquement orientées vers l'activation du système immunitaire cutané, et la stimulation des fonctions germinatives des cellules basales de l'épiderme à l'exclusion de tout effet secondaire sur l'immunité systémique (générale).

La présente invention a pour objet la réalisation de molécules peptidiques, homologues, ou dérivés des séquences peptidiques actives des principales hormones thymiques "la thymuline et la thymopoïétine".

Les nombreux essais cliniques effectués avec ces médiateurs hormonaux n'ont pas permis de mettre en évidence leur intérêt thérapeutique.

En effet, utilisés sous forme de préparations injectables pour le traitement des déficits immunitaires et des affections auto-immunes, les résultats ont tous été négatifs, en raison de leur activité ubiquitaire en fonction des doses administrées.

Il semble que ces effets ubiquitaires soient liés à la structure de ces composés, obtenu par synthèse, qui ne leur permet pas de se fixer normalement sur les globulines et les récepteurs cellulaires, compte tenu de leur administration parentérale.

C'est pourquoi l'un des objets de l'invention est la réalisation de composés sous forme de métallopeptides obtenu par synthèse, dont les activités physiologiques sont orientées vers la modulation du système immunitaire et l'activation des cellules germinatives de l'épiderme, par applications topiques locales, selon le principe pharmacologique des "relations-structures-activités" (Principe de Hânch).

Plus particulièrement la présente invention a pour objet une composition pharmaceutique ou cosmétologique contenant au moins un conjugué peptidique comprenant une séquence d'au moins trois acides aminés dérivés de la séquence totale de la thymuline ou de la thymopoïétine, les acides aminés pouvant être sous forme D, L, ou DL, ladite séquence étant conjuguées chimiquement ou physiquement avec au moins un composé sélectionné parmi :
- les acides monocarboxyliques de formule générale

   HOOC-R (I)

   dans laquelle R représente un radical aliphatique en C₁-C₂₀, linéaire ou ramifié, éventuellement substitué, pouvant comporter une ou plusieurs insaturations, ainsi que les dérivés alcool ou aldéhyde ou amide des acides de formule I ;
- les acides dicarboxyliques de formule générale

   HOOC-R₁-COOH (II)
dans laquelle R₁ représente un radical aliphatique divalent, comprenant au moins 3 atomes de carbone, de préférence 3 à 10 atomes de carbone, droit ou ramifié, notamment un radical alkyl, alkylène, alkénylène ou alkynylène, pouvant comporter une ou plusieurs insaturations éventuellement substitué, notamment par un ou plusieurs groupes amino, hydroxy, oxo ou un radical alkyl en C₁-C₃, R₁ pouvant en outre former un cycle avec l'une des fonctions acides.

Les conjugués selon l'invention, sont des dérivés de faible poids moléculaire, qui sont obtenus notamment sous forme de sels, d'esters ou d'amides des composés de formule I ou II.

Parmi les composés de formule I, sont particulièrement préférés des acides carboxyliques, à activité métabolique essentielle du cycle tricarboxylique de Krebs, agissant au niveau des mitochondries, comme coenzyme de décarboxylation oxydative, et dont les propriétés sont de se fixer préferentiellement par covalence sur les fonctions ε aminés de certains peptides, sous forme de lipo-amides, facilitant ainsi la présentation des conjugués peptidiques obtenus selon l'inventon au récepteur beta de classe G, des cellules de l'épiderme.

Pour ces acides, et d'autres convenant à la mise en oeuvre de l'invention, R peut représenter un radical aliphatique linéaire ou ramifié en C₁-C₂₀, notamment en C₁-C₄, substitué ou non, notamment un radical alkyl, alkényl ou alkynyl, pouvant comporter une ou plusieurs insaturations et pouvant être substitué par un ou plusieurs radicaux choisis dans le groupe comprenant :
NH₂, OH, oxo, thiol ou un radical cyclique non aromatique comportant de 5 à 6 atomes dans le cycle dont 1 ou 2 pouvant être différents du carbone, en particulier, S, O ou N, lesdits cycles pouvant être substitués par des radicaux alkyl en C₁ à C₃, notamment méthyl.

En particulier lorsque R représente une chaine aliphatique en C₁-C₂₀, elle peut être substituée par un cycle choisi parmi

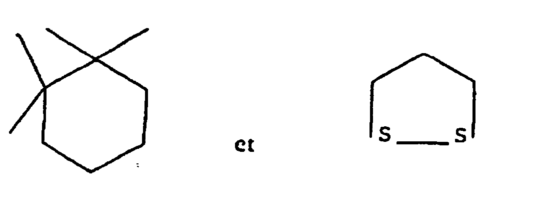

D'autres composés de formule I adaptés à la mise en oeuvre de l'invention sont ceux pour lesquels, dans la formule générale I, R peut représenter un radical monoinsaturé de configuration cis ou trans, de formule générale

R₂-CH=CH-

dans laquelle R₂ peut représenter un radical allyle linéaire ou ramifié comprenant au moins 6 atomes de carbone, de préférence 6 à 16 atomes de carbone, éventuellement substitué par un groupe amino, hydroxy ou oxo.

De manière avantageuse, dans la formule II, R₁ représente un reste alkylène en C₄-C₈ éventuellement substitué.

Des conjugués peptidiques selon l'invention sont notamment ceux pour lesquels l'acide de formule générale I est choisi parmi l'acide acétique, l'acide pyroglutamique, l'acide DL lipoïque, l'acide dihydrolipoïque, la N-lypoyl-lysine, les acides hydroxydécénoïques et décénoïliques, l'acide rétinoïque et ses dérivés, le rétinal et le rétinol, l'acide myristique et ses dérivés, l'acide palmitique, sous forme de sels, d'esters ou amides, ou bien l'acide de formule générale Il est choisi parmi l'acide adipique, l'acide α-amino adipique, l'acide pimélique, l'acide sébacique et leurs dérivés.

Les acides de formules générales I ou II sont de préférence choisis parmi l'acide acétique et ses dérivés, l'acide pyroglutamique, l'acide α-DL-Lipoïque et ses dérivés, l'acide dihydrolipoïque, la N-Lipoyl-Lysine, l'acide adipique, l'acide α-amino-adipique, l'acide pimélique, l'acide sébacique et ses dérivés, l'acide trans-10-hydroxy-Δ2-décénoïque et l'acide trans-oxo-9-decene-2-oïque, l'acide rétinoïque et ses dérivés, le rétinol, et le rétinal, l'acide myristique et l'acide palmitique.

La séquence en acides aminés des conjugués selon l'invention contiendra l'une des 3 séquences suivantes :

Gln-Gly-Gly

Gln-Gly-Gly

Lys-Asp-Val

En effet plus spéciquement, la présente invention concerne la réalisation de deux séries de molécules peptidiques dont les activités sont complémentaires, au niveau des applications dans les domaines de la médecine humaine et de la dermo-cosmétologie.

La présente invention concerne préférentiellement des conjugués peptidiques, dérivés de l'hormone thymique circulante, la thymuline, et dont les activités physiologiques sont orientées vers la maturation du système immunitaire et des différents médiateurs du système immunitaire cutané.

Ces conjugués peptidiques étant particulièrement orientés vers le traitement des maladies auto-immunes de la peau, et en particulier, la prévention et le traitement des alopécies quelles soient d'origine physiologique (vieillissement), traumatiques ou pathologiques.

La présente invention concerne également la réalisation de conjugués peptidiques, dérivés de l'hormone thymique cellulaire, la thymopoïétine, et dont les activités physiologiques sont également orientées vers la maturation du système immunitaire de la peau, tout en possédant une activité préférentielle pour la stimulation des métabolismes, des cellules de l'épiderme, en particulier des kératinocytes germinatifs, dont ils stimulent les sécrétions et les synthèses cndocellulaires des molécules de structures "kératines de bas poids moléculaires et kératohyalines".

Cette série de conjugués peptidiques étant particulièrement indiquée, vers le traitement des maladies de la peau, liées à des déficits immunitaires, et en particulier, au traitement préventif et curatif, du vieillissement cutané d'ordre physiologique (âge) ou pathologique.

De préférence la séquence en acides aminés des conjugués peptidiques dérivés de la thymuline répond à la formule générale suivante:

X-Gln-Gly-Gly-Y

dans laquelle Gln représente la glutamine ou un dérivé de la glutamine, Gly représente la glycine ou un dérivé de la glycine ; les dérivés comprennent notamment les dérivés halogénés des acides aminés en cause. Les acides aminés peuvent être sous forme naturelle ou non.

Selon l'un des aspects de l'invention, les conjugués peptidiques présenteront donc la formule suivante :

A-X-Gln-Gly-Gly-Y (III)

dans laquelle A est un composé de formule générale I ou II tel que défini précédemment, en particulier l'acide DL lipoique, l'acide dihydrolipoïque ou la N-lypoyllysine.
- X est : Ser, Lys-Ser, Ala-Lys-Ser, Pyr-Ala-Lys-Ser, une liaison ou Glx-Ala-Lys-Ser
   dans laquelle Glx est : Pyro-Glu, Glu ou Gly et ses dérivés
- Y est :

   Ser-Asn-OH

   Ser-Asn-NH2

   Ser-OH

   Ser-NH2

   Ser-NH2
Les amino-acides étant sous la forme L, D ou DL

Dans les formules qui suivent, Pyr représente l'acide pyroglutamique.

De préférence, les conjugués peptidiques de formule III comportent une séquence d'au moins 4 acides aminés, en particulier de 4 à 9 acides aminés, celle-ci comprenant avantageusement la séquence Gln-Gly-Gly-Ser.

La présente invention concerne tout particulièrement les conjugués suivants :

I A- Pyr - Ala - Lys - Ser - Gln - Gly - Gly - Ser - Asn - NH₂

II A- Pyr - Ala - Lys - Ser - Gln - Gly - Gly - Ser - NH₂

III A- Ala - Lys - Ser - Gln - Gly - Gly - Ser - Asn - NH₂

IV A- Ala - Lys- Ser - Gln Gly - Gly - Ser - NH₂

V A- Lys - Ser - Gln - Gly - Gly - Ser - Asn - NH₂

VI A- Lys - Ser - Gln - Gly - Gly - Ser - NH₂

VII A- Ser - Gln - Gly - Gly - Ser - Asn - NH₂

VIII A- Ser - Gln - Gly - Gly - Ser - NH₂

IX A- Gln - Gly - Gly - Ser - Asn - NH₂

X A- Gln - Gly - Gly - Ser - Asn - NH₂

X A- Gln - Gly - Gly - Ser - NH₂

XXIII A-Ala-Lys-Ser-Gln-Gly-Gly-Ser-Asn-OH.

A étant défini précédemment ainsi que les dérivés de ces molécules sous forme de sels, d'esters ou d'amides.

Les séquences d'amino-acides mentionnées ci-dessus, peuvent être des séquences d'amino-acides naturels ou non naturels.

II est également précisé que les conjugués peptidiques mentionnés ci-dessus et faisant l'objet de la présente invention, peuvent être obtenus sous la forme terminale NH₂ et sous la forme terminale OH.

De même, dans certains cas, il est possible que certains de ces amino-acides comportent des fonctions par exemple des glycosylations.

Selon un autre aspect, l'invention concerne des conjugués peptidiques dérivés de la thymopoiétine et répondant à la formule générale suivante :

A-W-Lys-Asp-Z (IV)

dans laquelle A est un composé de formule générale I ou II tel que défini précédemment ou le radical correspondant, et notamment l'acide acétique et ses dérivés, l'acide DL-α-lipoïque et ses dérivés, l'acide dihydrolipoïque, la N-lipoyl-lysine, l'acide adipique, l'acide α-amino-adipique, l'acide pimélique, l'acide sébacique et dérivés, l'acide trans-oxo-9-décéne-2-oïque, l'acide trans-hydroxy-10-décéne-2-oïque
- W représent :: Glu-Gln-Arg, Gln-Arg, Arg,
Arg-Lys-, Arg-Lys-Asp ou une liaison
- Z représente :: Val-Tyr-NH₂, Val-Tyr-OH
Val-NH₂, Val-OH, Tyr-OH, Tyr-NH₂,
OH ou NH₂
W et Z étant choisis de telle sorte que l'une au moins des séquences Arg-Lys-Asp ou Lys-Asp-Val est présente dans les composés de formule IV à l'exception des conjugués de formule XI, XII, XIII, XIV, XV et XVI tels que définis dans la revendication 1.

Des conjugués peptidiques de formule IV comportant la séquence Arg-Lys-Asp-Val donnent d'excellents résultats.

Les acides aminés peuvent être sous forme D, L ou DL et peuvent être éventuellement glycosylés.

Des conjugués peptidiques particulièrement adaptés, et dont les activités biologiques sont complémentaires des conjugués peptidiques de formule III, sont les conjugués de formule IV suivants :

XVII A-Lys-Asp-Val-Tyr-NH₂

XVIII A-Lys-Asp-Val-TyrOH

XIX A-Arg-Lys-Asp-Val-NH₂

XX A-Arg-Lys-Asp-Val-OH

XXI A-Arg-Lys-Asp-NH₂

XXII A-Arg-Lys-Asp-OH

"A" étant défini précédemment ainsi que les dérivés de ces molécules sous forme de sels, d'esters, ou d'amides.

Les séquences d'amino-acides mentionnés ci-dessus peuvent être des séquences d'amino-acides naturels ou non naturels.

Il est précisé que les conjugués peptidiques mentionnés ci-dessus peuvent être obtenus sous la forme terminale NH₂ ou OH.

Les conjugués peptidiques selon l'invention peuvent également se présenter sous forme de complexes moléculaires avec un métal, notamment le zinc.

Les conjugués selon l'invention pourront être obtenus par synthèse, par des techniques connues de l'homme du métier.

Les synthèses des peptides libres peuvent être effectuées d'après les techniques de MERRIFIELD, selon deux procédés.

Dans un premier procédé, on utilise un dérivé de la Glutamine protégé dans sa fonction d'amide γ par le "Mbh" 4,4-diméthoxybenzhydryl.

Dans un second procédé, la synthèse est effectuée sans protection de l'amide γ des résidus de la Glutamine.

En utilisant la première et la seconde procédure, la partie protégée C-terminale du dipeptide est associée respectivement au tétrapeptide protégé Ser-Gln-Gly et à l'hexapeptide protégé Ala-Lys-Gln-Gly-Gly.

Ainsi, la préparation des polypeptides de séquence

X-Gln-Gly-Gly-Y,

dans laquelle Y représente Ser-Asn et X représente Ser ou Ala-Lys-Ser, est effectuée en associant le dipeptide protégé Ser-Asn avec le peptide protégé X-Gln-Gly-Gly et par l'élimination consécutive possible des groupes protecteurs.

En utilisant la seconde procédure, c'est donc l'octapeptide Ala-Lys-Ser-Gln-Gly-Gly-Ser-Asn qui est obtenu, alors qu'en utilisant la première procédure, le même octapeptide est préparé à partir de l'hexapeptide Ser-Gln-Gly-Gly-Ser-Asn en passant par l'heptapeptide Lys-Ser-Gln-Gly-Gly-Ser-Asn.

L'octapeptide Ala-Lys-Ser-Gln-Gly-Gly-Ser-Asn permet alors de préparer les deux formes, par association du premier résidu PyroGlu ou Gln.

Le processus utilisé pour associer le résidu PyroGlu dans la séquence Ala-Lys-Ser-Gln-Gly-Gly-Y est le même quelles que soient les significations possibles de Y, à savoir Ser et Ser-Asn, et les significations possibles qui en sont dérivées par la modification d'un acide aminé, par exemple pour Ser-Asp, Ala-Asn.

Cette association est obtenue de manière avantageuse au moyen du dérivé PyroGlu-OTcp dans la séquence Ala-Lys-Ser-Gln-Gly-Gly-Y, dans laquelle certains acides aminés ont la possibilité d'être protégés dans leur fonction sur la chaîne latérale.

Les dérivés peptidiques faisant l'objet de la présente invention et précédemment décrits, peuvent être utilisés sous leur forme peptidique ou sous forme de conjugués, avec les acides carboxyliques précédemment décrits répondant à la formule générale I.

Ces mêmes conjugués peptidiques peuvent être utilisés sous forme de complexes moléculaires avec un métal.

Le métal utilisé pour la réalisation des complexes métallo-peptidiques de la présente invention est par exemple le Zinc, sous forme de ZnCl₂ qui peut être couplé sous forme de sel, avec un groupe carboxylique de l'amino acide terminal, ou sous forme de complexes "peptides-Zn²" dans les proportions suivantes de 0,5 à 5%, de préférence 1% de ZnCl₂ par molécule peptidique.

Ces proportions sont variables en fonction du poids moléculaire du conjugué peptidique.

Le complexe métallo-peptidique peut notamment être obtenu en solution à 10% de Zncl₂, pour une molécule peptidique, après chauffage à 20° C pendant 10 minutes.

Le complexe est ensuite purifié, par exemple sur colonne de biogel P-2, et élué avec de l'eau distillée et lyophilisé.

On obtient un colyophilisat stable contenant 1% de Zinc fixe à la molécule du peptide par affinité.

L'ensemble des composés selon la présente invention peut être utile à titre de médicament.

En particulier, les conjugués selon la présente invention peuvent servir à la préparation d'un médicament destiné à corriger les déficiences du système immunitaire.

Des compositions pharmaceutiques peuvent contenir au moins un conjugué selon l'invention et des excipients adaptés à l'administration par voie parentérale ou par voie topique externe.

Ces compositions sont utilisables aussi bien dans le domaine de la médecine humaine et vétérinaire, administrables par voie orale, ou parentérale, mais de préférence sous forme administrable par voie topique externe.

Des compositions particulièrement adaptées à la mise en oeuvre de l'invention sont celles contenant le conjugé suivant :

Pyr-Ala-Lys-Ser-Gln-Gly-Gly-Ser-Asn-OH

éventuellement sous forme de complexe avec un métal.

Les conjugués peptidiques faisant l'objet de l'invention, leurs dérivés et leurs compositons galéniques peuvent être présentés sous forme de crèmes, de laits, de lotions ou de sprays, et comporter des excipients connus et éventuellement d'autres principes actifs.

Ils peuvent être utilisés seuls, ou sous forme d'associations ou de compositions galéniques pour le traitement des maladies auto-immunes dans les différents domaines de la dermatologie.

L'invention concerne également l'utilisation des conjugués peptidiques en dermocosmétologie et en cosmétologie.

La présente invention a donc pour objet des compositions galéniques, pharmaceutiques, et cosmétologiques comprenant au moins un des conjugués peptidiques décrit précédemment.

Les conjugués peptidiques de l'invention, leurs dérivés, leurs associations avec des principes actifs connus, peuvent être utilisés sous forme de préparations cosmétologiques et capillaires, notamment pour le rajeunissement et le renouvellement des couches superficielles de la peau, et en particulier pour le traitement cosméto-capillaire de la chute des cheveux.

Les exemples qui suivent sont destinés à illustrer l'invention, et notamment l'activité immunostimulante des peptides thymiques de synthèse administrés par voie topique, sans aucunement en limiter la portée.

Dans ces exemples, on se référera aux figures suivantes:
**Figure 1** **:** Action de l'ETF sur la synthèse de DNA.
**Figure 2** **:** Action de l'ETF sur les cellules NK par voie topique (cellules cibles : YAC1). Le pourcentage de lyse est représenté aux jours 1, 3, 6, 9, 15, 18 et 21 de l'injection, pour les différents rapports cellules effectrices/cellules cibles.
**Figure 3** **:** Action d'ETF sur les cellules NK par voie topique (cellules cibles P815).
**Figure 4** **:** Action de l'ETF sur les cellules NK in vitro. Le pourcentage de lyse en fonction du rapport cellules effectrices/cellules cibles est donné pour différentes doses d'ETF.
**Figure 5** **:** Action de l'ETF sur les cellules NK par voie injectable. Le pourcentage de lyse en fonction du rapport cellules effectrices/cellules cibles est représenté après stimulation par 15 µg d'ETF, et pour des animaux témoins.
**Figure 6** **:** Rôle de l'interféron dans la stimulation des cellules NK par ETF par voie injectable. Le pourcentage de lyse est donné en fonction du rapport cellules effectrices/cellules cibles, en présence ou non d'anti-interféron.
**Figure 7** **:** Action de l'ETF sur des cellules NK par voie injectable : cinétique de la réponse 1, 3 et 8 jours après administration de 15 µg ETF par voie intra-péritonéale.

### EXEMPLE N° 1 - PREPARATION DU CONJUGUE N° II

A-Pyro-Ala-Lys-Ser-Gln-Gly-Gly-Ser-NH₂
A = acide DL Lipoïque

La synthèse est réalisée selon la méthodologie générale décrite selon les étapes suivantes :
1 - Boc-Gln-Gly-Gly-OMe
2 - Trifluoroacetate-Gln-Gly-Gly-OMe
3 - Z-Ser-NH-NH₂(But) -> Bop -> DIEA -> DMF
4 - Z-Ser-Glu-Gly-GLy-Ser-NH₂
5 - Acide Lipoïque -> BOP -> DIEA -> DMF
6 - Lipoyl-Pyro-Ala-Lys-Ser-Gln-Gly-Gly-Ser-NH₂
Analyse HPLC des amino-acides
Asp-1,02 ; Ser-1,77 ; Glu-1,0 ; Gly-2,00 ; Ala-0,91.

### EXEMPLE N° 2 - PREPARATION DU CONJUGUE N° III

A-Ala-Lys-Ser-Gln-Gly-Gly-Ser-Asn-NH₂
A = Acide Adipique

La synthèse a été réalisée selon la méthodologie générale selon les étapes suivantes :
1 - Z-D-Ala-Lys-(Boc)-Ser(But) -> DMF
2 - Lys-(Boc)-Ser(But)-Gln(Mbh) -> DMF
3 - Gly-Gly-Ser(But)-Asn-O-But
4 - Me.OH -> CH3COOH -> D-Ala-Lys(Boc)-Ser(But)-Gln(Mbh) -> Gly-Gly-Ser-Asn(But)
5 - Acide Adipique -> BOP -> DIEA
6 - Adipoyl-Ala-Lys-Ser-Gln-Gly-Gly-Ser-Asn-NH₂
Analyse HPLC des amino acides
Asp-1,02 ; Ser-1,77 ; Glu-1,0 ; Gly-2,00 ; Ala-0,91.

### EXEMPLE N° 3 - PREPARATION DU CONJUGUE N°I

A-Pyro-Ala-Lys-Ser-Gln-Gly-Gly-Ser-Asn-NH₂
A = Acide Acétique

Ce conjugué est obtenu par la méthodologie décrite à l'exemple 1 à partir du dérivé :
Ala-Lys(Boc)-Ser(But-Gln-Mbh)-Gly-Gly-Ser(But)-Asn(But)
décrit précédemment.

On obtient un produit homogène par chromatographie dans les solvants G et H, à pH 2, 3.
Analyse HPLC des amino acides.
Asp-0,97 ; Ser-1,67 ; Glu-1,84 ; Gly-2,00 ; Ala-0,98.

### EXEMPLE 4 - MESURE DE L'ACTIVITÉ SUR LES CELLULES DE LANGERHANS IA+ ET LES THYMOCYTES THY. 1.2+ DE L'ÉPIDERME DES SOURIS C57-BU6

### I Résumé

Les kératinocytes des souris "C57-BL/6" stimulés par les dérivés peptidiques des hormones thymiques libèrent les médiateurs (messagers cellulaires) tels que l'ETAF, l'IL1 et IL6, qui induisent une activation de la réponse immunitaire des cellules immunocompétentes de l'épiderme, les cellules de Langerhans la⁺ et les Thymocytes Thy 1.2⁺.

La mesure de cette activation par les techniques d'immunofluorescence est caractéristique de l'activité immunomodulatrice des peptides thymiques.

### II Matériel et Méthode

### 1 - Echantillons expérimentés - Code: ETF

En solution dans propylène glycol à (c) = 10⁻⁵ M/ml
ETF - Peptide - n° I
ETF - Peptide - n° III
ETF - Peptide - n° XV - exemple comparatif
ETF - Peptide - n° XIX
   témoin = propylène glycol

### 2 - Méthodologie

Des souris mâles C 57 - BU6 âgées de 5 à 7 semaines, réparties à raison de 4 parcage, avec libre accès à l'eau et à la nourriture soumises à une photopériode de 12 h de lumière par 24 heures, reçoivent quotidiennement et pendant 5 jours consécutifs une application topique du peptide dans du propylène glycol (vol de 50 µl), sur la face dorsale de l'oreille.

Le jour 6, les animaux sont sacrifiés. On procède au prélèvement des oreilles avec séparation de la face dorsale et de la face ventrale.

Les tissus de la face dorsale sont immergés dans de l'EDTA de Na (0,020 M) pendant 2 h à 37° C.

Après incubation, l'épiderme est prélevé sous forme de feuillet intact fixé à l'acétone et réhydraté dans du PBS (tampon phosphate).

Les CED (cellules épidermiques dendritiques) Thy 1-2 ⁺ et la ⁺ sont identifiées par double marquage en immunofluorescence indirecte.

Le nombre de cellules par mm² est déterminé par les zones périphériques de même surface, pour chaque feuillet épidermique.

Les CED (cellules épidermiques dendritiques) portant le marqueur la⁺ ont été identifiées par l'utilisation d'un anticorps monoclonal anti-la⁺.

Les feuillets épidermiques sont fixés à l'acétone, puis incubés pendant 1 h à 37° C en présence de l'anticorps monoclonal anti-la⁺ et marqués par la méthode de l'immunoperoxydase indirecte (Kit).

Après incubation pendant 10 minutes à 37° C avec une solution d'amino-éthyl-carbazol, la préparation est lavée au PBS.

Les CED Thy 1.2⁺ ont été identifiées par double marquage en immunofluorescence indirecte. Les feuillets épidermiques ont été fixés à l'acétone puis réhydratés dans le PBS.

Les tissus marqués simultanément par des anticorps de souris anti-Thy 1.2⁺ dilués au 1:100 pendant 16 h à 4° C. Les échantillons de tissus ont été lavés trois fois dans du PBS pendant 60 mn puis incubés 60 mn à 37° C, soit avec des anticorps de chèvre anti-souris couplés à la rhodamine, dilués au 1:20, soit avec des anticorps de chèvre anti-lapin couplés à la fluorescéine (fluorescine diluée au 1:20 dans le PBS. Les tissus ont ensuite été lavés dans le PBS avant d'être montés comme décrit précédemment).

Les témoins comportaient des feuillets épidermiques non marqués à l'anticorps primaire et incubés uniquement en présence des réactifs secondaires, afin de mettre en évidence d'éventuelles réactions croisées avec les anticorps conjugués à la rhodamine et à la fluorescéine.

Le nombre de cellules de Langerhans la⁺ ou de CED Thy 1.2⁺ par millimètre carré a été déterminé dans quatre zones périphériques de même surface pour chaque feuillet épidermique, par immunofluorescence, au microscope confocal Zeiss équipé à cet effet. Au moins quatre animaux ont été étudiés dans chacun des groupes. La densité de cellules de Langerhans la⁺ et de CED Thy 1.2 ⁺ est indiquée pour chaque mesure.

### 3 - Résultats

Les résultats résumés dans le tableau I montrent que ETF et ses homologues, administrés par voie topique à des souris C 57 BU6 augmentent de façon hautement significative, l'activité des cellules résidentes du système immunitaire cutané.

On observe notamment une augmentation significative des phénotypes des cellules dendritiques épidermiques de Langerhans la⁺ - (HLADR) à des doses de 0,1 à 1 nanogrammes.

Parallèlement, on observe également une augmentation significative de l'activité des thymocytes épidermiques de phénotype Thy 1.2 ⁺, c'est à dire ETAF dépendants, à des doses identiques.

La réponse immunitaire cutanée est sous la dépendance de l'activité des Kératinocytes nucléés qui sécrètent l'ETAF et les médiateurs de l'immunité, c'est à dire le 2ème signal sans lequel il n'y a pas de fonction immunitaire physiologique normale (Immuno-dermatologie L.D.H.). On peut considérer que l'ETF et ses homologues, rétablissent les fonctions immunitaires de l'épiderme par activation des Kératinocytes des couches basales de façon physiologique, par opposition à la stimulation antigènique exogène.

**Résultats exprimés sur 4 lots de 4 souris C 57 BL/6**

| Nombre de cellules Thy 1,2+ et la+ par mm2 d'épiderme | | | | | | | | | | | | | |
|---|---|---|---|---|---|---|---|---|---|---|---|---|---|
| Echantillons | Doses Nanog. | Cellules Thy 1,2+ | | | | Moyenne | % | Cellules la+ | | | | Moyenne | % |
| | | 1 | 2 | 3 | 4 | | T | 1 | 2 | 3 | 4 | | |
| Témoins PG | | 80 | 76 | 87 | 110 | 88,25 | | 98 | 102 | 100 | 110 | 102,50 | |
| | | | | | | | | | | | | | |
| ETF-I | 0,10 | 197 | 180 | 192 | 207 | 194,00 | 120 | 368 | 388 | 378 | 395 | 382,25 | 273 |
| | 0,50 | 200 | 208 | 199 | 219 | 206,50 | 134 | 360 | 392 | 386 | 399 | 384,25 | 275 |
| | 1,00 | 214 | 210 | 208 | 226 | 214,50 | 143 | 372 | 410 | 390 | 412 | 396,00 | 286 |
| | | | | | | | | | | | | | |
| ETF-III | 0,10 | 168 | 165 | 180 | 176 | 172,25 | 95 | 376 | 382 | 387 | 399 | 386,00 | 277 |
| | 0,50 | 190 | 190 | 205 | 200 | 196,25 | 122 | 380 | 388 | 299 | 388 | 363,73 | 255 |
| | 1,00 | 200 | 220 | 216 | 210 | 211,50 | 140 | 305 | 400 | 398 | 410 | 378,25 | 269 |
| | | | | | | | | | | | | | |
| ETF-XV exemple comparatif | 0,10 | 160 | 158 | 160 | 170 | 162,00 | 84 | 315 | 326 | 320 | 330 | 322,75 | 215 |
| | 0,50 | 176 | 168 | 170 | 186 | 175,00 | 98 | 305 | 345 | 330 | 345 | 331,25 | 223 |
| | 1,00 | 198 | 180 | 188 | 199 | 191,25 | 177 | 348 | 360 | 348 | 350 | 351,50 | 243 |
| | | | | | | | | | | | | | |
| ETF-XIX | 0,10 | 216 | 210 | 219 | 212 | 214,25 | 143 | 360 | 390 | 378 | 320 | 362,00 | 253 |
| | 0,50 | 220 | 215 | 228 | 216 | 219,75 | 149 | 388 | 398 | 386 | 400 | 393,00 | 283 |
| | 1,00 | 227 | 238 | 230 | 225 | 230,00 | 161 | 400 | 410 | 438 | 415 | 415,75 | 306 |

### EXEMPLE 5 - MESURE DE L'ACTIVITÉ ETF N° IX SUR LES KÉRATINOCYTES DE PEAU HUMAINE ISSUS DE PRIMOCULTURE

### I Matériel et Méthode

### 1 - Modèle Cellulaire

Culture des Kératinocytes humains normaux
- Sources : Plastie mammaire
- Primoculture : Les tissus fraîchement biopsiés, en asepsie totale, sont digérés à + 4° C dans la trypsine 0,25%, afin de séparer l'épiderme du derme.

L'épiderme est ensuite dissocié sous action mécanique douce ou enzymatique afin d'obtenir des cellules individualisées. Seules les cellules basales seront capables de se multiplier in vitro.

La culture est réalisée en athmosphère humide contrôlée (95% O₂ - 5% CO₂) à 37°C.

En culture monocouche, la concentration en calcium se situe entre 0,05 et 0,1 mM. Dans ces conditions, les Kératinocytes prolifèrent rapidement mais ne forment pas de strates. La synthèse des kératinocytes est maintenue. Les espaces intercellulaires sont larges : les connections cellulaires sont assurées par les microvillosités et non par les tight-junctions. Les tonofilaments de l'espace périnucléaire sont visibles mais ils ne sont pas encore rattachés aux plaques d'encrage.

La stratification peut être induite par une augmentation de la concentration finale en calcium dans le milieu de culture jusqu'à 1,2 mM. Alors la formation de desmosomes apparait au bout de 2 heures.
Le taux de sodium et de potassium intracellulaire augmente dès la 12ème heure, de plus, il n'est pas bloqué par les inhibiteurs classiques du flux Ca2⁺/Na⁺.

La stratification verticale est visible au bout de 2 jours et la différentiation terminale s'achève par des cellules à enveloppe cornifiée.

### (Hennings et coll. 1980).

### 2 - Echantillons analysés

I - Dérivé ETF n° IX, lyophilisé-dans Dextran (40g/I) 10⁻⁶ M dans milieu de culture final.
II - Thymopoïetine Crist. SIGMA - 10⁶ M dans milieu de culture final.
III - Témoin : Dextran 15.000 D - (40g/l).
IV - Protéines cellulaires - Sol. de broyat cellulaire de référence à 50 microgrammes par ml.

### 3 - Méthodologie

La culture de kératinocytes humains est réalisée comme décrit par Henning et coll (1983) ou Fuchs et coll (1981). On effectue également des cultures air-liquide selon la méthodologie suivante.

### - Lattices de collagène sur grille

Du collagène de type I ou IV est incorporé au milieu de culture.

Des fibroblastes 3T3 sont ajoutés à cette solution à la concentration de 1,5.10⁵/ml et ensemmencés en boite de pétri de 35 mm qui sont ensuite placées à 37° C.

Après la formation du gel, du milieu est rajouté de façon à recouvrir entièrement les lattices. Les kératinocytes sont ensemmencés et maintenus ainsi en culture immergée durant 7 jours.

A ce stade les lattices sont transférées sur des grilles en acier inoxydable. Ces grilles sont placées dans des boites contenant du milieu pendant 7 à 24 jours.

### - Membrane basale artificielle

Des cellules endothéliales de cornée de boeuf sont cultivées sur des filtres recouverts de collagène. Après stimulation avec un facteur de croissance type FGF, ces cellules sécrètent du collagène IV et de la laminine, ainsi que d'autres molécules qui se déposent sur le collagène reconstituant ainsi une pseudo-membrane basale.

Après élémination des cellules endothéliales bovines, les kératinocytes sont ensemmencés sur ce support.

### - Derme artificiel

Des fibroblastes sont incorporés dans un gel de collagène. Après rétraction du gel, les kératinocytes sont ensemmencés sur cette lattice.

### - Membrane basale du derme

Des fragments de peau humaine sont désépidermisés par immersion pendant 5 jours dans du PBS à 37° C. Après un tel traitement l'épiderme se détache complètement au dessus de la membrane basale. Des congélations et décongélations successives vont tuer les fibroblastes. Ce tissu, comportant le derme et la basale, servira de couche nourricière pour les kératinocytes.

| Culture sur : | | | Kéralohyaline | Kératine 67 kD |
|---|---|---|---|---|
| 1. | Plastique + immersion | | - | - |
| | | | | |
| 2. | Filtre couvert de coll. | I | - | - |
| | I ou IV | | | |
| | | E | - | - |
| | | | | |
| 3. | Filtre couvert de membrane basale artificielle | I | - | - |
| | | E | - | - |
| | | | | |
| 4. | Derme artificiel | I | ± | - |
| | | E | + | ++ |
| | | | | |
| 5. | Derme naturel désépidermisé et tué | I | ± | - |
| | | E | ++ | ++ |

| | | | | |
|---|---|---|---|---|
| I = Immergé E = Emergé | | | | |

Le contrôle de différenciation de l'épiderme est effectué par microscopie électronique (Prunieras, 1988), par analyse biochimique (Tinois et coll, 1993) et par analyse immunologique (Woodcock et coll, 1982, Prunieras, 1988). Cette dernière peut mettre en jeu des anticorps anti-involucrine, anti-transglutaminase épidermique, anti-fillagrine et anti-kératines (AE1, AE2, AE3).

Les kératines 48 Kd sont les marqueurs de la prolifération.

Les kératines 50 et 58 Kd sont les marqueurs permanents des kératines.

Les kératines 56,5 et 65/67 Kd sont les marqueurs de la différenciation.

Les marqueurs cellulaires choisis sont les Kératines de bas poids moléculaire 46/47 KD spécifiques de la croissance des cellules germinatives et de leur différentiation cellulaire.

Les filaggrines marqueurs de la Kératohyaline.

Marquage fluorescent indirect selon la technique de sandwich - 48 heures après l'apport de calcium au milieu de culture (stratification).

### I- Anticorps primaires "SIGMA"

- Ac de rat antikératine humaine 46-47 KD.
- Ac humain antikératine humaine 56,5/68 KD
- Ac humain antikératohyaline (Filaggrine)

### II - Anticorps secondaires "SIGMA"

- Ac de souris anti Ig G2 de rat marqués FITC.
- Ac de chèvre anti Ig G humaine marqué FITC.

### * Dosages microscopiques

- "Microscope ZEISS Confocal Laser Scanner"
- Epi-fluorescent - LSM 310
- Mesure des marqueurs cellulaires par immunofluorescence, avec enregistrement graphique.

### * Dosages spectrofluorimétriques

- "Spectrofluorimètre Perkin Elmer LS - 50 B".
- Sur broyat surnageants cellulaires.

### * Dosages des protéines cellulaires

- Technique de LOWRY au spectrophotomètre UV Perkin Elmer (surnageants de broyats cellulaires).

### II Résultats

Les tableaux n° II et III résument l'activité du peptide n° IX "ETF" sur la croissance des cellules germinatives des couches basales et suprobasales de l'épiderme - "Kératinocytes jeunes".

L'intensité de fluorescence des cellules traitées par ETF n°IX est apprécié comparativement à l'intensité de fluorescence des témoins.

On observe une augmentation significative des cellules pré-Kératinocytaires germinatives et de leur activité métabolique. En particulier au niveau des synthèses de Kératine de bas poids moléculaire et des Kératohyalines "Filaggrines", de structures semblables à celle de la medulla du thymus.

**Tableau Il**

| **"Résumé"** | | | | |
|---|---|---|---|---|
| **Action de l'ETF sur la synthèse kératinocytaire de la kératine 46-47 KD et des protéines cellulaires comparativement à un témoin (exprimés en microgrammes/ml).** | | | | |
| Produit | Concentration | Kératine/Protéine | Kératine/Protéine | Stimulation |
| | M | Moyenne | Ecart Type | % |
| Thymopoïétine | 10⁻⁶ | 94,8 | 3,4 | 19 |
| | 10⁻⁶ | 151,5 | 14,9 | 90 |
| ETF | 10⁻⁸ | 156,0 | 13,2 | 95 |
| N°-IX | 10⁻¹⁰ | 148,9 | 22,9 | 86 |
| | 10⁻¹² | 113,0 | 5,6 | 41 |

**Tableau III**

| **"Résumé"** | | | | |
|---|---|---|---|---|
| **Action de l'ETF sur la synthèse kératinocytaire de la kératohyaline et des protéines comparativement à un témoin (exprimés en microgrammes/ml).** | | | | |
| Produit | Concentration | Kératohyaline | Kératine /Protéine | Stimulation |
| | M | /Protéine | Ecart Type | % |
| | | Moyenne | | |
| Thymopoïétine | 10⁻⁶ | 4,99 | 0,54 | 35 |
| | 10⁻⁶ | 5,97 | 0,12 | 62 |
| ETF | 10⁻⁸ | 6,75 | 0,66 | 83 |
| N°-IX | 10⁻¹⁰ | 5,13 | 0,25 | 39 |
| | 10⁻¹² | 5,11 | 0,39 | 38 |

### EXEMPLE N° 6 - ACTION DE L'ETF SUR LES GRANULOCYTES "MONONUCLEAR PHAGOCYTE SYSTEM"

### I Introduction

La réponse immunitaire de l'Epiderme est sous la dépendance des Kératinocytes et des cellules de Langerhans, en association avec d'autres lignées cellulaires "granulocytes-thymocytes" etc...

Les cellules de Langerhans sont considérablement moins compétentes pour la phagocytose que les kératinocytes, Ce qui les différentie formellement des macrophages".

Elles sont semblables, phénotypiquement et fonctionnellement des cellules "voilées" (Veiled Cells) de la lymphe afférente et des cellules interdigitalisées des nodules lymphatiques.

L'action des cellules de Langerhans est le résultat d'une coopération cellulaire, en particulier avec les cellules granulocytaires "phagocytes" sous la dominante des kératinocytes.

Il était donc intéressant d'apprécier l'activité de l'ETF, sur l'activité phagocytaire des granulocytes polymorphonucléaires et des systèmes cellulaires voisins, notamment des cellules NK (ENKAF, Epidermal Cell derived Natural Killer Activating Factor).

### II - Action de l'ETF sur les granulocytes sanguins

### 1) Protocole:

Des cellules polymorphonucléaires de sang périphérique humain (granulocytes neutrophiles), à une concentration de 10⁶ cellules/ml, sont préincubées 20 mm à 37° C avant l'addition de luminolet de particules de zymosan opsonisées par du sérum.

La chimiluminescence est mesurée toutes les 3 minutes après l'addition du dernier réactif. Les résultats représentent la moyenne de 5 mesures indépendantes pour chaque point. (Déviation standard inférieure à 5% de moyenne).

Deux systèmes sont utilisés :

### A. L'agent inducteur est le peptide ETF n°1 présent pendant toute la durée de l'essai.

Des granulocytes normaux de sang périphérique humain, enrichis par sédimentation en dextrane et débarrassées des érythrocytes par lyse hypotonique. L'ETF est testé à des concentrations de 10, 1 et 0,1 µg/ml.

### B. L'agent inducteur ETF est éliminé par lavage après préincubation et avant l'addition des réactifs chimiluminescents.

Granulocytes normaux de sang périphérique humain purifiés par décantation sur dextrane et lyse hypotonique des érythrocytes. ETF est incubé 20 mm à 37° C avec les granulocytes puis éliminés par lavage avant l'addition de zymosan opsonisé et de luminol.

### 2 - Résultats : (les valeurs représentent la moyenne de 5 mesures)

| Système A | | | | | | | | |
|---|---|---|---|---|---|---|---|---|
| Traitement des cellules | | Valeurs de chimiluminescence à : (mm) | | | | | | |
| | | 3 | 6 | 9 | 12 | 15 | 18 | 21 |
| PBS | | 22 966 | 77 598 | 140 423 | 178 951 | 198 063 | 191 781 | 188 531 |
| ETF | 10µg/ml | 79 358 | 140 100 | 170 947 | 183 578 | 174 710 | 160 718 | 137 798 |
| | 1 µg/ml | 59 867 | 138 846 | 183 578 | 207 713 | 203449 | 188 901 | 168 813 |
| | 0,1µg/ml | 29 205 | 86 761 | 143 463 | 176 924 | 193 589 | 188 573 | 179 449 |

| Système B | | | | | | |
|---|---|---|---|---|---|---|
| Traitement des cellules | | Valeurs de chimiluminescence à : (mm) | | | | |
| | | 6 | 9 | 12 | 15 | 18 |
| PBS | | 69 924 | 114 751 | 144 83 8 | 162 594 | 164 750 |
| ETF | 10µg/ml | 103 105 | 119 677 | 125 307 | 119 952 | 112 499 |
| | 0,1 µg/ml | 100 532 | 124 819 | 134 788 | 129 950 | 133 707 |

### 3 - Conclusions :

A. ETF a un impact considérable sur la potentialisation de la chimiluminescence dès les premiers instants, à des concentrations de 10 et 1 µg/ml.
B. Même après lavage, ETF induit une activation significative des granulocytes à 10 et 0,1 µg/ml.

### EXEMPLE 7 - ACTION DE L'ETF SUR LA SYNTHÈSE DU DNA

### I - Protocole - Echantillon Peptide ETF n° VII

Un pool de cellules spléniques de souris normales est obtenu et incubé dans des microplaques avec ETF seul à des doses croissantes. Le DNA est dosé à J1, J3, J5.

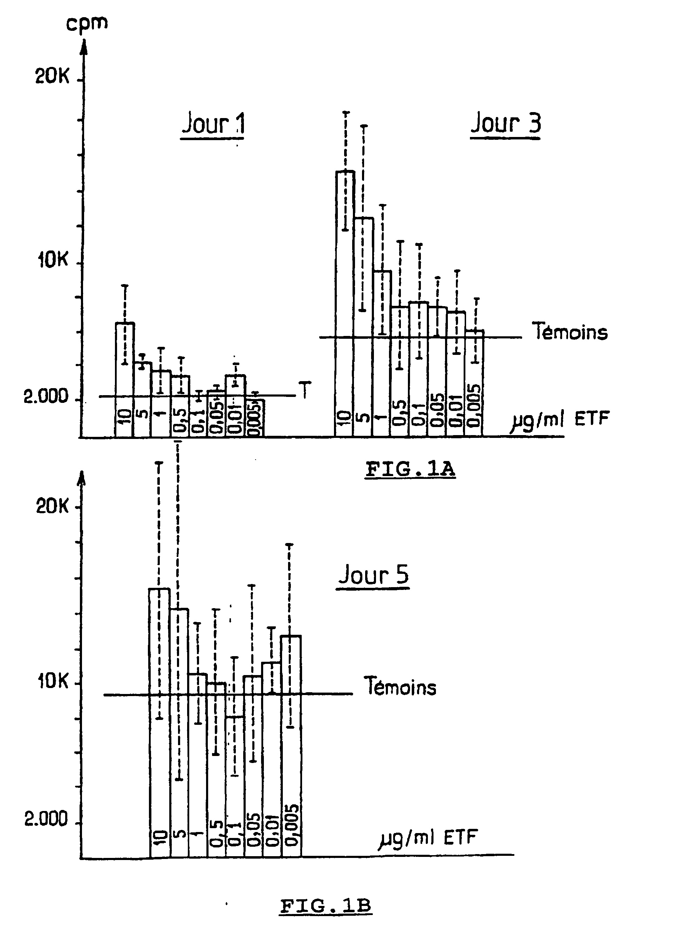

Les résultats sont présentés sur la figure 1.

### II Conclusions

L'ETF montre une réponse très nette, proportionnelle à la dose. La plus forte dose utilisée (10 µg/ml) donne la plus grande synthèse de DNA à tous les jours du contrôle avec un maximum au 3ème jour. Il faut noter, toutefois, que les valeurs de base sont supérieures au 3ème jour, la réponse spécifique est peut-être en réalité plus précoce.

### EXEMPLE 8 - ACTION D'ETF SUR LES CELLULES FORMANT DES PLAGES DE LYSE (PFC)

### I Protocole

50 souris Balb/C recevant 5 µg d'ETF par voie s.c. 10⁸ GRM (globules rouges de mouton) dans 0,1 ml de PBS sont injectés par voie i.v. 20, 10, et 3 jours après ETF ou 2 jours auparavant. Technique PFC de Cunningham et Szenberg.

### II Résultats

Nombre de PFC pour 10⁶ cellules spléniques.

| Traitement | Date d'immunostimulation | Nombre de PFC à | | | |
|---|---|---|---|---|---|
| | | J+3 | J+4 | J+5 | J+6 |
| Témoins GRM | 0 | 86 | 509 | 374 | 119 |
| ETF5 µg s.c. | J-20 | 69 | 438 | 318 | 84 |
| | J-10 | 323 | 823 | 337 | 104 |
| | J-3 | 57 | 399 | 353 | 129 |
| | J+2 | 119 | 410 | 336 | 135 |

### III Conclusions :

On observe une réponse qui présente un maximum à J + 4 avec une augmentation importante, très significative, du nombre de PFC chez les animaux ayant reçu de l'ETF 10 jours avant l'antigène.

### EXEMPLE 9 - ACTION D'EVF SUR LES CELLULES NK

### I Action d'ETF sur les cellules NK - voie topique

### 1 - Protocole général voie topique :

souris CBA/H (90 souris)
cellules cibles :
* YAC-1 sensibles aux NK
* P 815 non sensibles aux NK
* ETF n° VII

Les souris reçoivent chaque jour par voie topique 15 µg d'ETF en solution dans du Propylène Glycol à 10⁻⁵ M/ml, pendant 14 jours consécutifs. Une dose identique de rappel est administrée le 18ème jour.

La mesure de l'activité lytique est faite tous les 3 jours par comptage du ⁵¹Cr libéré pour des rapports cellules effectrices : cellules cibles de 200 : 1, 100 : 1, 50 : 1, 25 : 1, pendant 4 heures d'incubation.

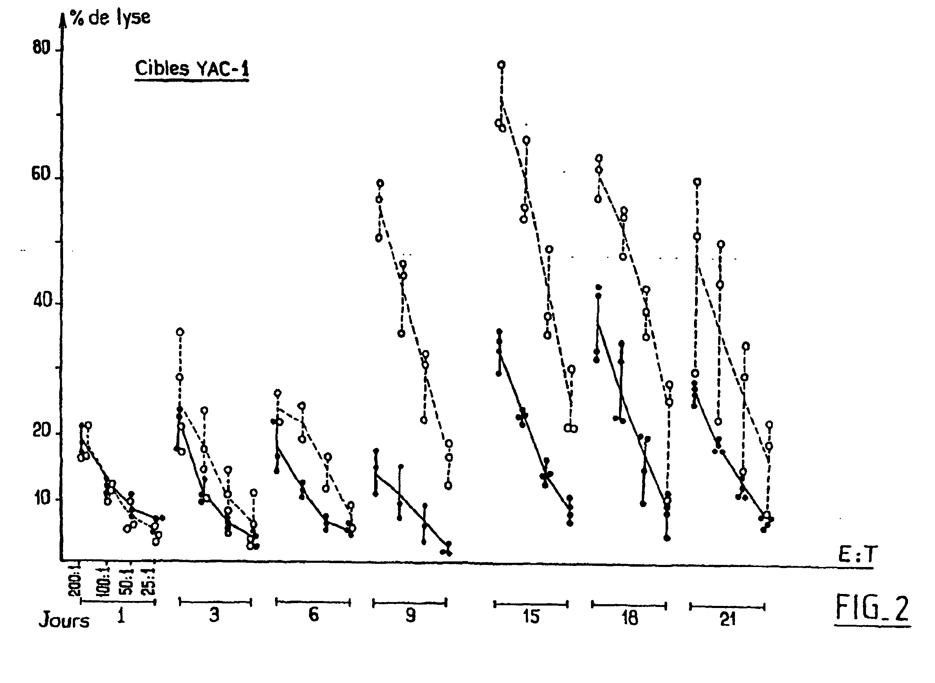

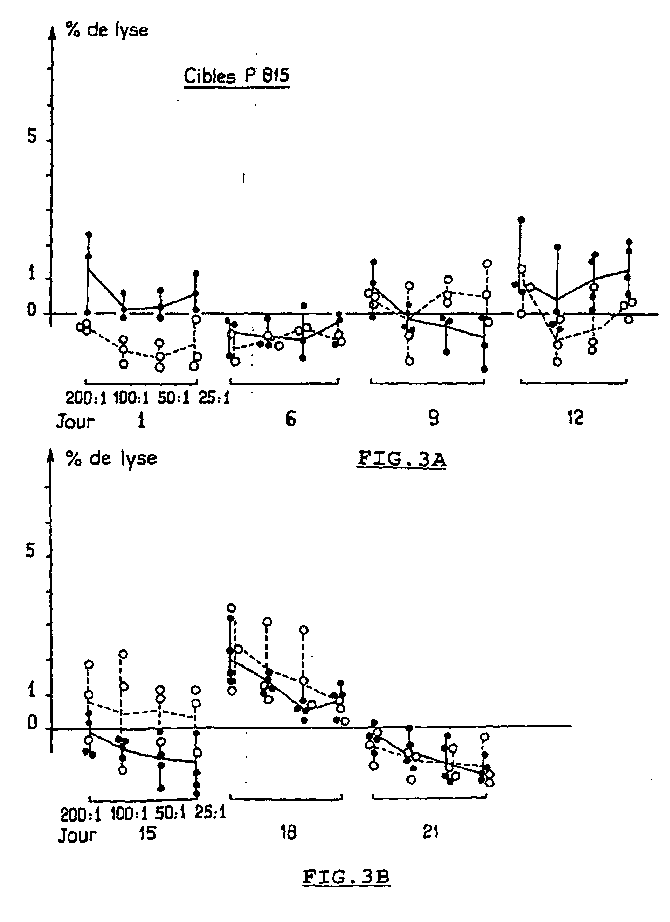

Les résultats sur les cellules NK par voie topique sont présentés sur les figures 2 et 3.

Les résultats sont extrêment nets. On constate, en fonction du temps, après le début du traitement par ETF, une augmentation hautement significative de l'activité NK. Cette activité qui est décelable dès le 3ème jour devient très importante au 6ème et au 9ème jour et se maintient à un niveau très élevé pendant toute la durée de l'expérimentation (21 jours).

### 2 - Conclusions

Aucune cytotoxicité non spécifique sur les cellules P 815 n'est décelable à aucun moment de l'expérimentation. Ceci exclut que l'administration de l'ETF conduise à l'induction de cellules de nature cytolytique non spécifique ou à l'activation polyclonale des cellules T cytolytiques.

### II Action d'ETF sur les cellules NK - In vitro

### 1 - Protocole de l'essai "in vitro"

Cellules spléniques normales de souris CBA. Incubateur à 37° C + CO2 10⁷ cellules par ml de milieu RPMI 1640 + 5% de sérum foetal de veau. Incubation avec des quantités variables de la substance à étudier. Cellules cibles YAC-1.

La mesure de la lyse est faite par comptage du ⁵¹Cr libéré pour des rapports cellules effectrices/cellules cibles de 200 : 1, 100 : 1, 50 : 1, 25 : 1 pendant 4 heures d'incubation.

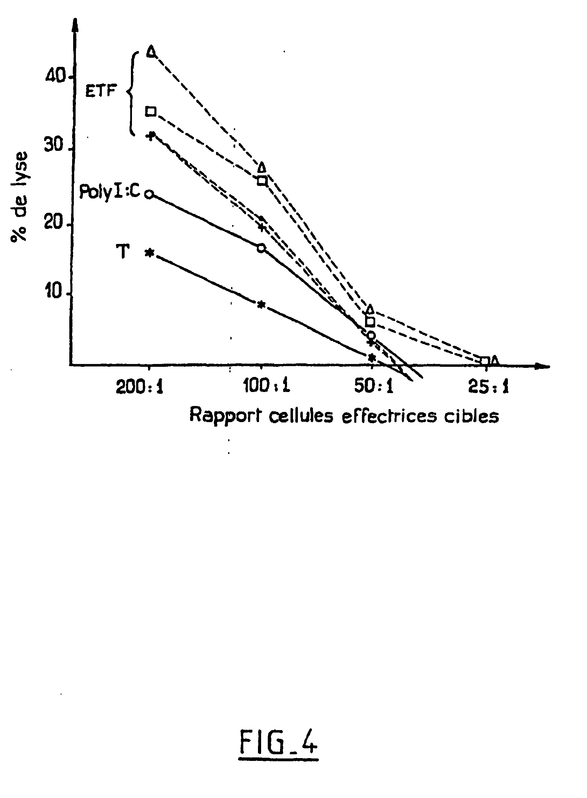

Les résultats sont présentés sur la figure 4.

### 2 - Conclusions :

ETF stimule très fortement l'activité des cellules NK in vitro de façon proportionnelle à la dose.

A une concentration de 0,1 µg/ml, l'activité de l'ETF est encore très supérieure à 25 µg/ml de poly I : C.

(Activité au moins 100 fois supérieure à celle du produit de référence poly I : C, inducteur d'interféron).

### III Action d'ETF sur les cellules NK - Voie injectable

### 1 - Protocole général voie injectable:

Souris CBA/H de 4 à 5 mois. (140 souris) cellules cibles :
* YAC-1 sensibles aux NK (lymphome induit/virus moloney)
* P 815 insensibles aux NK (DBA/2 mastocytoma)

La substance à tester (ETF n°VII) est injectée par voie i.p. dans 0,2 ml de PBS, 24 heures avant le test. La mesure de la lyse est faite par comptage du 51 Cr libéré pour des rapports cellules effectrices : cibles de 200 : 1,100 : 1, 50 : 1, 25 : 1 pendant 4 heures d'incubation.

### 2 - Résultats NK/voie injectable

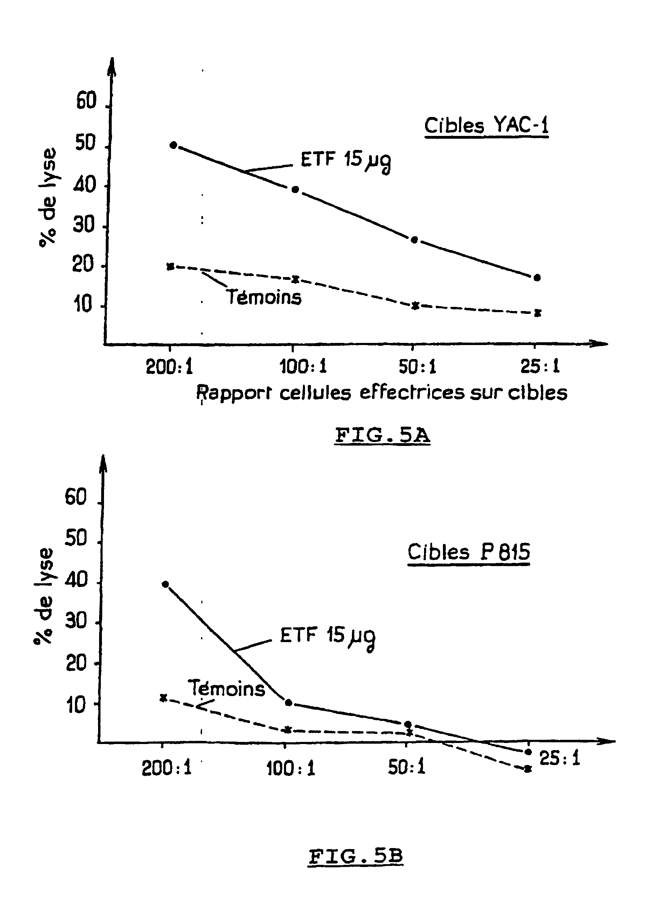

Les résultats de l'activité de stimulation des cellules NK par ETF sont représentés sur la figure 5. Ils montrent une forte activation des cellules NK, très significative (P<0,01) chez les animaux ayant reçu l'ETF (15 mg/i.p.) par rapport aux témoins. Pas de cytotoxicité non spécifique sur les P815.

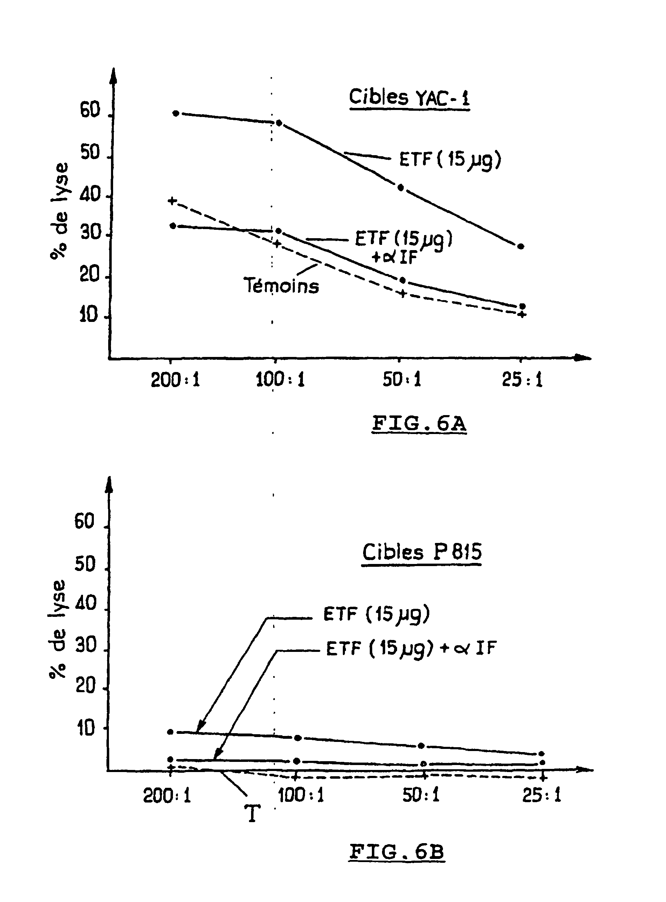

Sur la figure 6, on voit que l'activation des cellules NK par ETF est due à un effet inducteur d'interféron. On observe une réduction drastique des cellules NK lors de l'administration simultanée d'anti-interféron (α-IF).

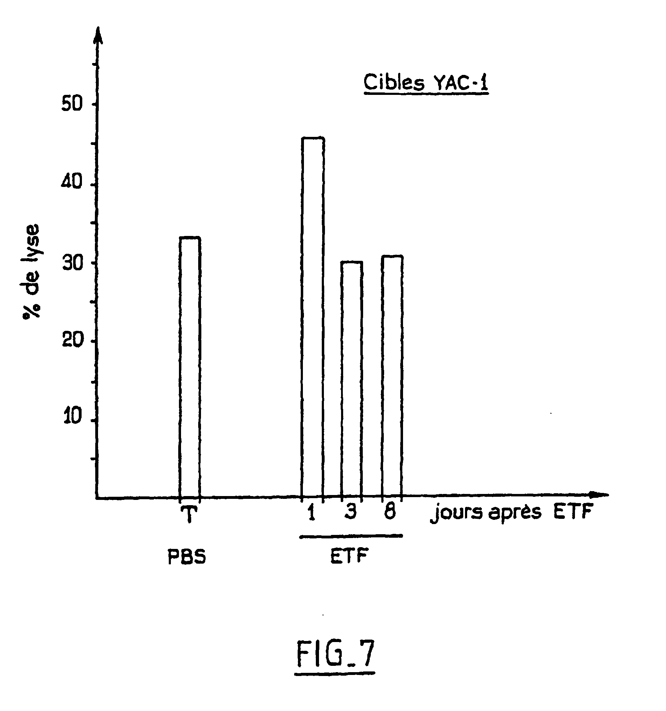

La cinétique de la réponse des cellules NK aux jours 1, 3 ou 8 de l'injection, par rapport à un témoin PBS, est représentée sur la figure 7. La stimulation des cellules NK par EFT est de courte durée par voie injectable.

Chez les animaux jeunes (7 à 9 mois), ETF stimule à la fois les cellules NK et pré-NK de façon identique à des doses de tilorone de 1 mg par animal.

### LEGENDES DES FIGURES

Figure 1 :
   ETF. Synthèse du DNA
Figure 2 :
   ETF. Cellules NK/voie topique

   ○-----○ traités/ETF
   •___• témoins
Figure 3 :
   ETF. Cellules NK/voie topique

   •___• témoins
   ○-----○ traités
Figure 4 :
   ETF : cellules NK 'in vitro'
   Comparaison ETF 0,1 --> 100 µg/ml
   avec Poly 1 : C 25 µg/ml

   ○__○__ Poly 1 : C 25 µg/ml (témoin +)
   *__*__ PBS (témoin -)
   •-----•-- ETF 0.1 µg/ml
   +-----+-- FTF 1,0 µg/ml
   □-----□-- ETF 10,0 µg/ml
   Δ-----Δ-- ETF 100,0 µg/ml
Figure 5:
   ETF. Cellules NK voie injectable
Figure 6 :
   ETF. Cellules NK voie injectable
   Rôle de l'interféron dans la stimulation des cellules NK par ETF
Figure 7 :
   ETF. Cellules NK voie injectable
   Cinétique de la réponse NK ETF
   Test NK 1, 3 ou 8 jours après 15 µg ETF voie (i.p.)

### BIBLIOGRAPHIE

E TINOIS, H. DUMAS, Q, GAETANI, D. DUPONT, X.POURADIER DUTEIL, A. ROUGIER Nouv. Dermatol. 12 n° 7, 510, 1993.
H. HENNING, K.A. HOLBROOK, S.H. YUSPA The J. of Invest. Dermatol. 81 50-55, 1983.
E. FUCHS, H. GREEN CELL, 25 617-625,1981.
M. PRUNIERAS Méthodes in vitro en pharmaco-toxicologie, Colloque INSERM 170 67-76, 1988.
J. WOODCOCK-MITCHELL, R. EICHNER, W.G. NELSON, T. SUN The J. of Cell Biol., 95 580-588, 1992.

## Revendications

1. Composition pour l'administration par voie topique, **caractérisée en ce que** qu'elle contient
(i) au moins un conjugué peptidique, qui comprend une séquence d'au moins 3 acides aminés dérivés d'une hormone thymique choisie parmi la thymuline et la thymopoïétine, les acides aminés pouvant être indépendamment sous forme D, L ou DL,
ladite séquence étant conjuguée chimiquement ou physiquement avec au moins un composé sélectionné parmi :
- les acides monocarboxyliques de formule générale
HOOC-R (I)
dans laquelle R représente un radical aliphatique en C₁-C₂₀, linéaire ou ramifié, éventuellement substitué, pouvant comporter une ou plusieurs insaturations, ainsi que les dérivés alcool ou aldéhyde ou amide des acides de formule I ;
- les acides dicarboxyliques de formule générale
HOOC-R₁-COOH (II)
dans laquelle R₁ représente un radical aliphatique divalent, comprenant au moins 3 atomes de carbone, de préférence 3 à 10 atomes de carbone, droit ou ramifié, notamment un radical alkyl, alkylène, alkénylène ou alkynylène, pouvant comporter une ou plusieurs insaturations éventuellement substitué, notamment par un ou plusieurs groupes amino, hydroxy, oxo ou un radical alkyl en C₁-C₃, R₁ pouvant former un cycle avec l'une des fonctions acides,
(ii) en combinaison avec un excipient pharmaceutiquement et/ou cosmétologiquement acceptable, adapté à l'administration par voie topique externe,
et **en ce que** le conjugué présente la formule générale
A-X-Gln-Gly-Gly-Y (III)
dans laquelle
A est un composé de formule générale I ou II ou le radical correspondant
- X est : Ser, Lys-Ser, Ala-Lys-Ser, Pyr-Ala-Lys-Ser, une liaison ou Glx-Ala-Lys-Ser
dans laquelle Glx est : Pyro-Glu, Glu ou Gly et ses dérivés
- Y est : Ser-Asn-OH
Ser-Asn-NH₂
Ser-OH
Ser-NH₂
les acides aminés étant sous forme D, L ou DL ou le conjugué présente la formule générale
A-W-Lys-Asp-Z (IV)
dans laquelle
A est un composé de formule générale I ou II ou le radical correspondant
W représente : Glu-Gln-Arg, Gln-Arg, Arg,
Arg-Lys-, Arg-Lys-Asp ou une liaison
Z représente : Val-Tyr-NH₂, Val-Tyr-OH
Val-NH₂, Val-OH, Tyr-OH, Tyr-NH₂,
OH ou NH₂
l'une au moins des séquences Arg-Lys-Asp ou Lys-Asp-Val étant présente,
les acides aminés étant sous forme D, L ou DL,
à l'exception des conjugués peptidiques de formule suivante :
XI A-Glu-Gln-Arg-Lys-Asp-Val-Tyr-NH₂
XII A-Glu-Gln-Arg-Lys-Asp-Val-Tyr-OH
XIII A-Gln-Arg-Lys-Asp-Val-Tyr-NH₂
XIV A-Gln-Arg-Lys-Asp-Val-Tyr-OH
XV A-Arg-Lys-Asp-Val-Tyr-NH₂
XVI A-Arg-Lys-Asp-Val-Tyr-OH

2. Composition selon la revendication 1, **caractérisée en ce que** dans la formule générale I, R peut représenter :
- un radical monoinsaturé de configuration cis ou trans, de formule générale
R₂-CH=CH-
dans laquelle R₂ peut représenter un radical alkyle linéaire ou ramifié comprenant au moins 6 atomes de carbone, de préférence 6 à 16 atomes de carbone éventuellement, substitué par un groupe amino, hydroxy ou oxo ;
- un radical aliphatique linéaire ou ramifié en C₁-C₂₀ substitué ou non, notamment un radical alkyl, alkényl ou alkynyl pouvant comporter une ou plusieurs insaturations et pouvant être substitué par un ou plusieurs radicaux choisis dans le groupe comprenant :
NH2, OH, oxo, thiol ou un radical cyclique non aromatique comportant de 5 à 6 atomes dans le cycle dont 1 ou 2 pouvant être différents du carbone, en particulier, S, O ou N, lesdits cycles pouvant être substitués par des radicaux alkyl en C₁ à C₃, notamment méthyle.

3. Composition selon l'une des revendications 1 ou 2, **caractérisée en ce que** dans la formule II, R₁ représente un reste alkylène en C₄-C₈ éventuellement substitué.

4. Composition selon l'une des revendications 1 à 3, **caractérisée en ce que** les acides de formule générale I ou II sont choisis parmi l'acide acétique et ses dérivés, l'acide pyroglutamique, l'acide DL lipoïque et ses dérivés, l'acide dihydrolipoïque et dérivés, la N-Lipoyl-Lysine, l'acide adipique, l'acide α-amino-adipique, l'acide pimélique, l'acide sébacique et dérivés, les acides gras α-mono-insaturés pour lesquels "R₂" représente un reste alkyle, linéaire ou ramifié en C₇, en particulier les acides hydroxydécénoïques et décénoïliques.

5. Composition selon la revendication 4, **caractérisée en ce que** les acides de formule générale I ou II sont de préférence choisis parmi l'acide acétique et ses dérivés, l'acide pyroglutamique, l'acide DL-Lipoïque et ses dérivés, l'acide dihydrolipoïque, l'acide N-Lipoyl-Lysine, l'acide adipique, l'acide α-amino-adipique, l'acide pimélique, l'acide sébacique et ses dérivés, l'acide trans-10-hydroxy-A2-décénoïque et l'acide trans-oxo-9-décénoïque.

6. Composition selon l'une des revendications 1 à 5, **caractérisée en ce que** la séquence d'aminoacides est liée à l'acide ou au dérivé de formule I ou II, sous forme de sel, d'ester ou d'amide.

7. Composition selon l'une des revendications 1 à 6, **caractérisée en ce que** le conjugué est sélectionné parmi les dérivés peptidiques suivants :
I A- Pyr - Ala - Lys - Ser - Gln - Gly - Gly - Ser - Asn - NH₂
II A- Pyr - Ala - Lys - Ser - Gln - Gly - Gly - Ser - NH₂
III A- Ala - Lys - Ser-Gln - Gly - Glu - Ser - Asn - NH₂
IV A- Ala - Lys - Ser - Gln - Gly - Gly - Ser - NH₂
V A- Lys - Ser - Gln - Gly - Gly - Ser - Asn - NH₂
VI A- Lys - Ser - Gln - Gly - Gly - Ser - NH₂
VII A- Ser - Gln - Gly - Gly - Ser - Asn - NH₂
VIII A- Ser - Gln - Gly - Gly - Ser - NH₂
IX A- Gln - Gly - Gly - Ser - Asn - NH₂
X A- Gln - Gly - Gly - Ser - NH₂
XVII A-Lys-Asp-Val-Tyr- NH₂
XVIII A-Lys-Asp-Val-Tyr-OH
XIX A-Arg-Lys-Asp-Val- NH₂
XX A-Arg-Lys-Asp-Val-OH
XXI A-Arg-Lys-Asp- NH₂
XXII A-Arg-Lys-Asp-OH
XXIII A-Ala-Lys-Ser-GIn-Gly-Gly-Ser-Asn-OH
A étant un composé de formule 1 ou II, ou le radical correspondant,
les acides aminés pouvant être sous forme D, L, ou DL.

8. Composition selon l'une des revendications 1 à 7, **caractérisée en ce qu'**au moins une partie des acides aminés est sous forme glycosylée.

9. Composition selon l'une des revendications 1 à 8, **caractérisée en ce qu'**au moins un conjugué est sous forme de complexe métallopeptidique lié chimiquement ou physiquement à un sel de Zinc.

10. Composition selon l'une des revendications 1 à 9, **caractérisée en ce qu'**elle contient le conjugué suivant :
Pyr-Ala-Lys-Ser-Gln-Gly-Gly-Ser-Asn-OH

11. Utilisation d'une composition selon l'une des revendications 1 à 10 pour la préparation d'un médicament destiné à corriger les déficiences du système immunitaire.

12. Utilisation d'un conjugué selon l'une des revendications 1 à 10 pour la préparation d'une composition destinée au traitement et à la prévention de la chute des cheveux.

13. Composition selon l'une des revendications 1 à 10, **caractérisée en ce qu'**il s'agit d'une préparation à usage dermatologique, utilisable sous forme de crèmes, de laits, de lotions, de sprays, en administration topique pour le traitement préventif et curatif des alopécies.

14. Composition selon l'une des revendications 1 à 10, **caractérisée en ce qu'**il s'agit d'une composition dermocosmétologique.

15. Composition selon l'une des revendications 1 à 10 ou 14, **caractérisée en ce qu'**il s'agit d'une préparation dermocosmétologique ou cosmétique, à l'usage des soins de beauté et du rajeunissement cutané en applications locales, utilisables sous forme de crèmes, de gels, de laits, de lotions et de sprays.

16. Composition selon l'une des revendications 1 à 10 ou 14, **caractérisée en ce qu'**il s'agit de préparations cosméto-capillaires destinées à la prévention et au traitement de la chute des cheveux, applicables localement sous forme de lotions, de sprays, et d'ampoules lyophilisées, avec solvant, pour applications locales.

17. Conjugué peptidique susceptible d'entrer dans une composition selon l'une des revendications précédentes, **caractérisé en ce qu'**il comprend une séquence d'au moins 3 acides aminés dérivés de la thymuline, les acides aminés pouvant être indépendamment sous forme D, L ou DL,
ladite séquence étant conjuguée chimiquement ou physiquement avec au moins un composé sélectionné parmi :
- les acides monocarboxyliques de formule générale
HOOC-R (I)
dans laquelle R représente un radical aliphatique en C₁-C₂₀, linéaire ou ramifié, éventuellement substitué, pouvant comporter une ou plusieurs insaturations, ainsi que les dérivés alcool ou aldéhyde ou amide des acides de formule I ;
- les acides dicarboxyliques de formule générale
HOOC-R₁-COOH (II)
dans laquelle R₁ représente un radical aliphatique divalent, comprenant au moins 3 atomes de carbone, de préférence 3 à 10 atomes de carbone, droit ou ramifié, notamment un radical alkyl, alkylène, alkénylène ou alkynylène, pouvant comporter une ou plusieurs insaturations éventuellement substitué, notamment par un ou plusieurs groupes amino, hydroxy, oxo ou un radical alkyl en C₁-C₃, R₁ pouvant former un cycle avec l'une des fonctions acides,
et **en ce qu'**il présente la formule générale
A-X-Gln-Gly-Gly-Y (III)
dans laquelle
A est un composé de formule générale I ou II ou le radical correspondant
- X est : Ser, Lys-Ser, Ala-Lys-Ser, Pyr-Ala-Lys-Ser, une liaison ou Glx-Ala-Lys-Ser
dans laquelle Glx est : Pyro-Glu, Glu ou Gly et ses dérivés
- Y est : Ser-Asn-OH
Ser-Asn- NH₂
Ser-OH
Ser- NH₂
les acides aminés étant sous forme D, L ou DL.

18. Conjugué peptidique selon la revendication 17, **caractérisé en ce que** dans la formule générale I, R peut représenter :
- un radical monoinsaturé de configuration cis ou trans, de formule générale
R₂-CH=CH-
dans laquelle R₂ peut représenter un radical alkyle linéaire ou ramifié comprenant au moins 6 atomes de carbone, de préférence 6 à 16 atomes de carbone éventuellement, substitué par un groupe amino, hydroxy ou oxo ;
- un radical aliphatique linéaire ou ramifié en C₁-C₂₀ substitué ou non, notamment un radical alkyl, alkényl ou alkynyl pouvant comporter une ou plusieurs insaturations et pouvant être substitué par un ou plusieurs radicaux choisis dans le groupe comprenant :
NH2, OH, oxo, thiol ou un radical cyclique non aromatique comportant de 5 à 6 atomes dans le cycle dont 1 ou 2 pouvant être différents du carbone, en particulier, S, O ou N, lesdits cycles pouvant être substitués par des radicaux alkyl en C₁ à C₃, notamment méthyle,
et dans la formule générale II, R₁ représente un reste alkylène en C₄-C₈, éventuellement substitué.

19. Conjugué selon l'une des revendications 17 ou 18, **caractérisé en ce que** les acides de formule générale I ou II sont choisis parmi l'acide acétique et ses dérivés, l'acides pyroglutamique, l'acide DL lipoïque et ses dérivés, l'acide dihydrolipoïque et dérivés, la N-Lipoyl-Lysine, l'acide adipique, l'acide α-amino-adipique, l'acide pimélique, l'acide sébacique et ses dérivés, les acides gras α-mono-insaturés pour lesquels "R₂" représente un reste alkyle, linéaire ou ramifié en C₇, en particulier les acides hydroxydécénoïques et décénoïliques, tel que l'acide trans-10-hydroxy-Δ2-décénoïque et l'acide trans-oxo-9-décénoïque.

20. Conjugué selon l'une des revendications 17 à 19, **caractérisé en ce que** la séquence d'aminoacides est liée à l'acide ou au dérivé de formule I ou II, sous forme de sel, d'ester ou d'amide.

21. Conjugué selon l'une des revendications 17 à 20, **caractérisé en ce qu'**il est sélectionné parmi les dérivés peptidiques suivants :
I A - Pyr - Ala - Lys - Ser - Gln - Gly - Gly - Ser - Asn - NH₂
II A - Pyr - Ala - Lys - Ser - Gln - Gly - Gly - Ser - NH₂
III A - Ala - Lys - Ser - Gln - Gly - Gly - Ser - Asn - NH₂
IV A - Ala - Lys - Ser - Gln - Gly - Gly - Ser - NH₂
V A - Lys - Ser - Gln - Gly - Gly - Ser - Asn - NH₂
VI A - Lys - Ser - Gln - Gly - Gly - Ser - NH₂
VII A - Ser - Gln - Gly - Gly - Ser - Asn - NH₂
VIII A - Ser - Gln - Gly - Gly - Ser - NH₂
IX A - Gln - Gly - Gly - Ser - Asn - NH₂
X A - Gln - Gly - Gly - Ser - NH₂
XXIII A-Ala-Lys-Ser-Gln-Gly-Gly-Ser-Asn-OH ,
A étant un composé de formule I ou II, ou le radical correspondant, les acides aminés pouvant être sous forme D, L, ou DL, une partie des acides aminés pouvant être sous forme glycosylée.

22. Conjugué selon l'une des revendications 17 à 21, **caractérisé en ce qu'**il est sous forme de complexe métallo-peptidique lié chimiquement ou physiquement à un sel de zinc.

23. A titre de médicament, conjugué selon l'une des revendications 17 à 22.

## Claims

1. Composition for topical-route administration,
**characterized in that** it comprises
(i) at least one peptide conjugate which includes a sequence of at least 3 amino acids which are derived from a thymic hormone which is selected from thymulin and thymopoietin, with it being possible for the amino acids to be independently in D, L or DL form,
the said sequence being conjugated chemically or physically with at least one compound which is selected from:
- monocarboxylic acids of the general formula
HOOC-R (I)
in which R represents a straight-chain or branched, optionally substituted C₁-C₂₀ aliphatic radical which can contain one or more unsaturations,
as well as the alcohol or aldehyde or amide derivatives of the acids of the formula I;
- dicarboxylic acids of the general formula
HOOC-R₁-COOH (II)
in which R₁ represents a straight-chain or branched divalent aliphatic radical which comprises at least 3 carbon atoms, preferably from 3 to 10 carbon atoms, in particular an alkyl, alkylene, alkenylene or alkynylene radical, and which can contain one or more unsaturations and can optionally be substituted, in particular by one or more amino, hydroxyl or oxo groups or by a C₁-C₃ alkyl radical, with it also being possible for R₁ to form a cycle with one of the acid functions,
(ii) in combination with a pharmaceutically and/or cosmetologically acceptable excipient which is suitable for being administered by an external topical route, and **in that** the conjugate has the general formula
A-X-Gln-Gly-Gly-Y (III)
in which A is a compound of the general formula I or II or the corresponding radical
- X is: Ser, Lys-Ser, Ala-Lys-Ser, Pyr-Ala-Lys-Ser,
a bond or Glx-Ala-Lys-Ser
in which Glx is: Pyro-Glu, Glu or Gly and its derivatives
- Y is: Ser-Asn-OH
Ser-Asn-NH₂
Ser-OH
Ser-NH₂
with the amino acids being in the D, L or DL form, or the conjugate has the general formula
A-W-Lys-Asp-Z (IV)
in which
A is a compound of the general formula I or II or the corresponding radical
W represents: Glu-Gln-Arg, Gln-Arg, Arg,
Arg-Lys-, Arg-Lys-Asp or a bond
Z represents: Val-Tyr-NH₂, Val-Tyr-OH
Val-NH₂, Val-OH, Tyr-OH, Tyr-NH₂,
OH or NH₂
with at least one of the sequences Arg-Lys-Asp and Lys-Asp-Val being present and with the amino acids being in the D, L or DL form,
with the exception of the peptide conjugates of the following formula:
XI A-Glu-Gln-Arg-Lys-Asp-Val-Tyr-NH₂
XII A-Glu-Gln-Arg-Lys-Asp-Val-Tyr-OH
XIII A-Gln-Arg-Lys-Asp-Val-Tyr-NH₂
XIV A-Gln-Arg-Lys-Asp-Val-Tyr-OH
XV A-Arg-Lys-Asp-Val-Tyr-NH₂
XVI A-Arg-Lys-Asp-Val-Tyr-OH

2. Composition according to Claim 1, **characterized in that**, in the general formula I, R can represent:
- a monounsaturated radical having the cis or trans configuration, of the general formula
R₂-CH=CH-
in which R₂ can represent a straight-chain or branched alkyl radical which comprises at least 6 carbon atoms, preferably from 6 to 16 carbon atoms, and which is optionally substituted by an amino, hydroxyl or oxo group;
- a substituted or unsubstituted, straight-chain or branched C₁-C₂₀ aliphatic radical, in particular an alkyl, alkenyl or alkynyl radical, which can contain one or more unsaturations and which can be substituted by one or more radicals which are selected from the group comprising:
NH₂, OH, oxo or thiol, or a nonaromatic cyclic radical which contains from 5 to 6 atoms in the cycle, 1 or 2 of which can be different from carbon, in particular S, O or N, with it being possible for the said cycles to be substituted by C₁ to C₃ alkyl radicals, in particular methyl.

3. Composition according to Claim 1 or 2, **characterized in that**, in the formula II, R₁ represents an optionally substituted C₄-C₈ alkylene radical.

4. Composition according to one of Claims 1 to 3, **characterized in that** the acids of the general formula I or II are selected from acetic acid and its derivatives, pyroglutamic acid, DL-lipoic acid and its derivatives, dihydrolipoic acid and derivatives, N-lipoyllysine, adipic acid, α-aminoadipic acid, pimelic acid, sebacic acid and derivatives, and the α-monounsaturated fatty acids in which "R₂" represents a straight-chain or branched C₇ alkyl radical, in particular the hydroxy-decenoic and decenoic acids.

5. Composition according to Claim 4, **characterized in that** the acids of the general formula I or II are preferably selected from acetic acid and its derivatives, pyroglutamic acid, DL-lipoic acid and its derivatives, dihydrolipoic acid, N-lipoyllysine, adipic acid, α-aminoadipic acid, pimelic acid, sebacic acid and its derivatives, trans-10-hydroxy-Δ2-decenoic acid and trans-9-oxodecenoic acid.

6. Composition according to one of Claims 1 to 5, **characterized in that** the amino acid sequence is linked to the acid or to the derivative of the formula I or II in salt, ester or amide form.

7. Composition according to one of Claims 1 to 6, **characterized in that** the conjugate is selected from the following peptide derivatives:
I A- Pyr - Ala - Lys - Ser - Gln - Gly - Gly - Ser - Asn - NH₂
II A- Pyr - Ala - Lys - Ser - Gln - Gly - Gly - Ser - NH₂
III A- Ala - Lys - Ser-Gln - Gly - Glu - Ser - Asn - NH₂
IV A- Ala - Lys - Ser - Gln - Gly - Gly - Ser - NH₂
V A- Lys - Ser - Gln - Gly - Gly - Ser - Asn - NH₂
VI A- Lys - Ser - Gln - Gly - Gly - Ser - NH₂
VII A- Ser - Gln - Gly - Gly - Ser - Asn - NH₂
VIII A- Ser - Gln - Gly - Gly - Ser - NH₂
IX A- Gln - Gly - Gly - Ser - Asn - NH₂
X A- Gln - Gly - Gly - Ser - NH₂
XVII A-Lys-Asp-Val-Tyr- NH₂
XVIII A-Lys-Asp-Val-Tyr-OH
XIX A-Arg-Lys-Asp-Val- NH₂
XX A-Arg-Lys-Asp-Val-OH
XXI A-Arg-Lys-Asp- NH₂
XXII A-Arg-Lys-Asp-OH
XXIII A-Ala-Lys-Ser-GIn-Gly-Gly-Ser-Asn-OH
with A being a compound of the formula I or II or the corresponding radical, and with it being possible for the amino acids to be in D, L or DL form.

8. Composition according to one of Claims 1 to 7, **characterized in that** at least one part of the amino acids is in glycosylated form.

9. Composition according to one of Claims 1 to 8, **characterized in that** at least one conjugate is in the form of a metallopeptide complex linked chemically or physically to a zinc salt.

10. Composition according to one of Claims 1 to 9, **characterized in that** it comprises the following conjugate:
Pyr-Ala-Lys-Ser-Gln-Gly-Gly-Ser-Asn-OH.

11. Use of a composition according to one of Claims 1 to 10 for preparing a medicament which is intended to correct deficiencies of the immune system.

12. Use of a conjugate according to one of Claims 1 to 10 for preparing a composition which is intended for treating and preventing hair loss.

13. Composition according to one of Claims 1 to 10, **characterized in that** it is a preparation for dermatological use, which preparation can be used, in the form of creams, milks, lotions or sprays, in topical administration for the prophylactic and therapeutic treatment of alopecias.

14. Composition according to one of Claims 1 to 10, **characterized in that** it is a dermocosmetological composition.

15. Composition according to one of Claims 1 to 10 or 14, **characterized in that** it is a dermocosmetological or cosmetic preparation which is for beauty care and skin rejuvenation use in local applications and which can be used in the form of creams, gels, milks, lotions and sprays.

16. Composition according to one of Claims 1 to 10 or 14, **characterized in that** the preparations are cosmeto-capillary preparations which are intended for preventing and treating hair loss and which can be applied locally in the form of lotions, sprays and lyophilized ampoules, with solvent, for local applications.

17. Peptide conjugate which can form part of a composition according to one of the preceding claims, **characterized in that** it includes a sequence of at least 3 amino acids which are derived from thymulin, with it being possible for the amino acids to be independently in D, L or DL form,
with the said sequence being conjugated chemically or physically with at least one compound which is selected from:
- the monocarboxylic acids of the general formula
HOOC-R (I)
in which R represents an optionally substituted straight-chain or branched C₁-C₂₀ aliphatic radical which can include one or more unsaturations,
as well as the alcohol or aldehyde or amide derivatives of the acids of the formula I;
- the dicarboxylic acids of the general formula
HOOC-R₁-COOH (II)
in which R₁ represents a straight-chain or branched divalent aliphatic radical which contains at least 3 carbon atoms, preferably from 3 to 10 carbon atoms, in particular an alkyl, alkylene, alkenylene or alkynylene radical, which can include one or more unsaturations and which can be optionally substituted, in particular by one or more amino, hydroxyl or oxo groups or by a C₁-C₃ alkyl radical, with it being possible for R₁ to form a cycle with one of the acid functions,
and **in that** it has the general formula
A-X-Gln-Gly-Gly-Y (III)
in which
A is a compound of the general formula I or II or the corresponding radical
- X is: Ser, Lys-Ser, Ala-Lys-Ser, Pyr-Ala-Lys-Ser, a bond or Glx-Ala-Lys-Ser
in which Glx is: Pyro-Glu, Glu or Gly and its derivatives
- Y is: Ser-Asn-OH
Ser-Asn-NH₂
Ser-OH
Ser-NH₂
with the amino acids being in D, L or DL form.

18. Peptide conjugate according to Claim 17, **characterized in that**, in the general formula I, R can represent:
- a monounsaturated radical having the cis or trans configuration, of the general formula
R₂-CH=CH-
in which R₂ can represent a straight-chain or branched alkyl radical which contains at least 6 carbon atoms, preferably from 6 to 16 carbon atoms, and which is optionally substituted by an amino, hydroxyl or oxo group;
- a substituted or unsubstituted, straight-chain or branched C₁-C₂₀ aliphatic radical, in particular an alkyl, alkenyl or alkynyl radical, which can contain one or more unsaturations and which can be substituted by one or more radicals which are selected from the group comprising:
NH₂, OH, oxo or thiol or a nonaromatic cyclic radical which contains from 5 to 6 atoms in the cycle, 1 or 2 of which can be different from carbon, in particular S, O or N, with it being possible for the said cycles to be substituted by C₁ to C₃ alkyl radicals, in particular methyl,
and, in the general formula II, R₁ represents an optionally substituted C₄-C₈ alkylene radical.

19. Conjugate according to one of Claims 17 or 18, **characterized in that** the acids of the general formula I or II are selected from acetic acid and its derivatives, pyroglutamic acid, DL-lipoic acid and its derivatives, dihydrolipoic acid and derivatives, N-lipoyllysine, adipic acid, a-aminoadipic acid, pimelic acid, sebacic acid and its derivatives, the a-monounsaturated fatty acids in which "R₂" represents a straight-chain or branched C₇ alkyl radical, in particular the hydroxy-decenoic and decenoic acids, such as trans-10-hydroxy-Δ2-decenoic acid and trans-9-oxodecenoic acid.

20. Conjugate according to one of Claims 17 to 19, **characterized in that** the amino acid sequence is linked to the acid or to the derivative of the formula I or II, in salt, ester or amide form.

21. Conjugate according to one of Claims 17 to 20, **characterized in that** it is selected from the following peptide derivatives:
I A - Pyr - Ala - Lys - Ser - Gln - Gly - Gly - Ser - Asn - NH₂
II A - Pyr - Ala - Lys - Ser - Gln - Gly - Gly - Ser - NH₂
III A - Ala - Lys - Ser - Gln - Gly - Gly - Ser - Asn - NH₂
IV A - Ala - Lys - Ser - Gln - Gly - Gly - Ser - NH₂
V A - Lys - Ser - Gln - Gly - Gly - Ser - Asn - NH₂
VI A - Lys - Ser - Gln - Gly - Gly - Ser - NH₂
VII A - Ser - Gln - Gly - Gly - Ser - Asn - NH₂
VIII A - Ser - Gln - Gly - Gly - Ser - NH₂
IX A - Gln - Gly - Gly - Ser - Asn - NH₂
X A - Gln - Gly - Gly - Ser - NH₂
XXIII A-Ala-Lys-Ser-Gln-Gly-Gly-Ser-Asn-OH
with A being a compound of the formula I or II, or the corresponding radical, with it being possible for the amino acids to be in D, L or DL form, and with it being possible for one part of the amino acids to be in glycosylated form.

22. Conjugate according to one of Claims 17 to 21, **characterized in that** it is in metallopeptide complex form linked chemically or physically to a zinc salt.

23. Conjugate according to one of Claims 17 to 22, as a medicament.

## Patentansprüche

1. Zusammensetzung zur Verabreichung auf topischem Wege, **dadurch gekennzeichnet, dass** sie enthält
(i) wenigstens ein Peptidkonjugat, das eine Sequenz von wenigstens 3 Aminosäuren, abgeleitet von einem aus Thymulin und Thymopoetin ausgewählten Thymushormon, umfasst, wobei die Aminosäuren unabhängig voneinander in D-, L- oder DL-Form vorliegen können,
wobei die Sequenz chemisch oder physikalisch mit wenigstens einer Verbindung konjugiert ist, die ausgewählt ist unter:
- den Monocarbonsäuren der allgemeinen Formel
HOOC-R (I),
in welcher R für einen linearen oder verzweigten, gegebenenfalls substituierten aliphatischen C₁-C₂₀-Rest, der eine oder mehrere Ungesättigtheiten enthalten kann, steht,
wie auch den Alkohol- oder Aldehyd- oder Amidderivaten der Säuren der Formel I;
- den Dicarbonsäuren der allgemeinen Formel
HOOC-R₁-COOH (II),
in welcher R₁ für einen gerad- oder verzweigtkettigen zweiwertigen aliphatischen Rest, der wenigstens 3 Kohlenstoffatome, vorzugsweise 3 bis 10 Kohlenstoffatome umfasst, insbesondere einen Alkyl-, Alkylen-, Alkenylen- oder Alkinylenrest, der eine oder mehrere Ungesättigtheiten umfassen kann, gegebenenfalls substituiert ist, insbesondere durch eine oder mehrere Amino-, Hydroxy-, Oxogruppen oder einen C₁-C₃-Alkylrest, wobei R₁ mit einer der Säurefunktionen einen Cyclus bilden kann, steht,
(ii) in Kombination mit einem pharmazeutisch und/oder aus kosmetischer Hinsicht verträglichen Träger, der für die Verabreichung auf topischem äußerlichem Wege angepasst ist,
und **dadurch**, dass das Konjugat die allgemeine Formel
A-X-Gln-Gly-Gly-Y (III)
aufweist, in der
A eine Verbindung der allgemeinen Formel I oder II oder der entsprechende Rest ist,
- X : Ser, Lys-Ser, Ala-Lys-Ser, Pyr-Ala-Lys-Ser, eine Bindung oder Glx-Ala-Lys-Ser ist, worin Glx: Pyro-Glu, Glu oder Gly und dessen Derivate ist,
- Y : Ser-Asn-OH
Ser-Asn-NH₂
Ser-OH
Ser-NH₂ ist,
wobei die Aminosäuren in D-, L- oder DL-Form vorliegen,
oder das Konjugat die allgemeine Formel
A-W-Lys-Asp-Z (IV)
aufweist, in der
A eine Verbindung der allgemeinen Formel I oder II oder der entsprechende Rest ist, W für: Glu-Gln-Arg, Gln-Arg, Arg,
Arg-Lys-, Arg-Lys-Asp oder eine Bindung steht,
Z für : Val-Tyr-NH₂, Val-Tyr-OH,
Val-NH₂, Val-OH, Tyr-OH, Tyr-NH₂,
OH oder NH₂ steht,
wobei wenigstens eine der Sequenzen Arg-Lys-Asp oder Lys-Asp-Val vorhanden ist,
wobei die Aminosäuren in D-, L- oder DL-Form vorliegen,
mit der Ausnahme der Peptidkonjugate mit der folgenden Formel:
XI A-Glu-Gln-Arg-Lys-Asp-Val-Tyr-NH₂
A-Glu-GlXII n-Arg-Lys-Asp-Val-Tyr-OH
XIII A-Gln-Arg-Lys-Asp-Val-Tyr-NH₂
XIV A-Gln-Arg-Lys-Asp-Val-Tyr-OH
XV A-Arg-Lys-Asp-Val-Tyr-NH₂
XVI A-Arg-Lys-Asp-Val-Tyr-OH.

2. Zusammensetzung nach Anspruch 1, **dadurch gekennzeichnet, dass** in der allgemeinen Formel I R stehen kann für:
- einen einfach ungesättigten Rest von cis- oder trans-Konfiguration mit der allgemeinen Formel
R₂-CH=CH-,
in welcher R₂ für einen linearen oder verzweigten Alkylrest, der wenigstens 6 Kohlenstoffatome, vorzugsweise 6 bis 16 Kohlenstoffatome umfasst und der gegebenenfalls durch eine Amino-, Hydroxy- oder Oxogruppe substituiert ist, steht;
- einen substituierten oder nicht-substituierten, linearen oder verzweigten aliphatischen C₁-C₂₀-Rest, insbesondere einen Alkyl-, Alkenyl- oder Alkinylrest, der eine oder mehrere Ungesättigtheiten umfassen kann und substituiert sein kann durch einen oder mehrere Reste, die ausgewählt sind aus der Gruppe, umfassend:
NH₂, OH, Oxo, Thiol oder einen cyclischen, nicht-aromatischen Rest, der 5 bis 6 Atome im Ring umfassen kann, von denen 1 oder 2 von Kohlenstoff verschieden sein können, insbesondere S, O oder N sein können, wobei die Ringe durch C₁-C₃-Alkylreste, insbesondere Methylreste, substituiert sein können.

3. Zusammensetzung nach einem der Ansprüche 1 oder 2, **dadurch gekennzeichnet, dass** in der Formel II R₁ für einen gegebenenfalls substituierten C₄-C₈-Alkylenrest steht.

4. Zusammensetzung nach einem der Ansprüche 1 bis 3, **dadurch gekennzeichnet, dass** die Säuren der allgemeinen Formel I oder II unter Essigsäure und deren Derivaten, Pyroglutaminsäure, DL-Liponsäure und deren Derivaten, Dihydroliponsäure und Derivaten, N-Lipoyllysin, Adipinsäure, α-Aminoadipinsäure, Pimelinsäure, Sebacinsäure und Derivaten, den α-mono-ungesättigten Fettsäuren, für die "R₂" einen linearen oder an C7 verzweigten Alkylrest darstellt, insbesondere den Hydroxydecensäuren und Decenoylsäuren, ausgewählt sind.

5. Zusammensetzung nach Anspruch 4, **dadurch gekennzeichnet, dass** die Säuren der allgemeinen Formel I oder II vorzugsweise unter Essigsäure und deren Derivaten, Pyroglutaminsäure, DL-Liponsäure und deren Derivaten, Dihydroliponsäure, N-Lipoyllysinsäure, Adipinsäure, α-Aminoadipinsäure, Pimelinsäure, Sebacinsäure und deren Derivaten, trans-10-Hydroxy-Δ2-decensäure und trans-Oxo-9-decensäure ausgewählt sind.

6. Zusammensetzung nach einem der Ansprüche 1 bis 5, **dadurch gekennzeichnet, dass** die Aminosäuresequenz an die Säure oder an das Derivat der Formel I oder II in Form von Salz, Ester oder Amid gebunden ist.

7. Zusammensetzung nach einem der Ansprüche 1 bis 6, **dadurch gekennzeichnet, dass** das Konjugat unter den folgenden Peptidderivaten ausgewählt ist:
I A- Pyr - Ala - Lys - Ser - Gln - Gly - Gly - Ser - Asn - NH₂
II A- Pyr - Ala - Lys - Ser - Gln - Gly - Gly - Ser - NH₂
III A- Ala - Lys - Ser-Gln - Gly - Glu - Ser - Asn - NH₂
IV A- Ala - Lys - Ser - Gln - Gly - Gly - Ser - NH₂
V A- Lys - Ser - Gln - Gly - Gly - Ser - Asn - NH₂
VI A- Lys - Ser - Gln - Gly - Gly - Ser - NH₂
VII A- Ser - Gln - Gly - Gly - Ser - Asn - NH₂
VIII A- Ser - Gln - Gly - Gly - Ser - NH₂
IX A- Gln - Gly - Gly - Ser - Asn - NH₂
X A- Gln - Gly - Gly - Ser - NH₂
VXII A-Lys-Asp-Val-Tyr- NH₂
XVIII A-Lys-Asp-Val-Tyr-OH
XIX A-Arg-Lys-Asp-Val- NH₂
XX A-Arg-Lys-Asp-Val-OH
XXI A-Arg-Lys-Asp- NH₂
XXII A-Arg-Lys-Asp-OH
XXIII A-Ala-Lys-Ser-GIn-Gly-Gly-Ser-Asn-OH,
wobei A eine Verbindung der Formel I oder II oder der entsprechende Rest ist,
wobei die Aminosäuren in D-, L- oder DL-Form vorliegen können.

8. Zusammensetzung nach einem der Ansprüche 1 bis 7, **dadurch gekennzeichnet, dass** wenigstens ein Teil der Aminosäuren in glycosylierter Form vorliegt.

9. Zusammensetzung nach einem der Ansprüche 1 bis 8, **dadurch gekennzeichnet, dass** wenigstens ein Konjugat in Form eines Metall-Peptid-Komplexes vorliegt, der chemisch oder physikalisch an ein Zinksalz gebunden ist.

10. Zusammensetzung nach einem der Ansprüche 1 bis 9, **dadurch gekennzeichnet, dass** sie das folgende Konjugat enthält:
Pyr - Ala - Lys - Ser - Gln - Gly - Gly - Ser - Asn - OH.

11. Verwendung einer Zusammensetzung nach einem der Ansprüche 1 bis 10 zur Herstellung eines Arzneimittels, das dazu bestimmt ist, Mängel des Immunsystems zu korrigieren.

12. Verwendung eines Konjugats nach einem der Ansprüche 1 bis 10 zur Herstellung einer Zusammensetzung, die für die Behandlung und die Verhütung von Haarausfall bestimmt ist.

13. Zusammensetzung nach einem der Ansprüche 1 bis 10, **dadurch gekennzeichnet, dass** es sich um ein Präparat für eine dermatologische Verwendung, das in Form von Cremes, Milchpräparaten, Lotionen, Sprays verwendet werden kann, im Rahmen einer topischen Verabreichung für die präventive und heilende Behandlung von Alopezien handelt.

14. Zusammensetzung nach einem der Ansprüche 1 bis 10, **dadurch gekennzeichnet, dass** es sich um eine kosmetische Zusammensetzung für die Haut handelt.

15. Zusammensetzung nach einem der Ansprüche 1 bis 10 oder 14, **dadurch gekennzeichnet, dass** es sich um ein kosmetisches Präparat für die Haut oder kosmetisches Präparat für eine Verwendung bei der Schönheitspflege und der Verjüngung der Haut im Rahmen von lokalen Anwendungen handelt, das in Form von Cremes, Gelpräparaten, Milchpräparaten, Lotionen und Sprays verwendet werden kann.

16. Zusammensetzung nach einem der Ansprüche 1 bis 10 oder 14, **dadurch gekennzeichnet, dass** es sich um kosmetische Präparate für das Haar handelt, die für die Verhütung und die Behandlung von Haarausfall bestimmt sind, die lokal in Form von Lotionen, Sprays und lyophilisierten Ampullen, mit Lösemittel, für lokale Anwendungen aufgetragen werden können.

17. Peptidkonjugat, das in der Lage ist, in eine Zusammensetzung nach einem der vorangegangenen Ansprüche Eingang zu finden, **dadurch gekennzeichnet, dass** es eine Sequenz von wenigstens 3 Aminosäuren, abgeleitet von Thymulin, umfasst,
wobei die Aminosäuren unabhängig voneinander in D-, L- oder DL-Form vorliegen können,
wobei die Sequenz chemisch oder physikalisch mit wenigstens einer Verbindung konjugiert ist, die ausgewählt ist unter:
- den Monocarbonsäuren der allgemeinen Formel
HOOC-R (I),
in welcher R für einen linearen oder verzweigten, gegebenenfalls substituierten aliphatischen C₁-C₂₀-Rest, der eine oder mehrere Ungesättigtheiten enthalten kann, steht,
wie auch den Alkohol- oder Aldehyd- oder Amidderivaten der Säuren der Formel I;
- den Dicarbonsäuren der allgemeinen Formel
HOOC-R₁-COOH (II),
in welcher R₁ für einen gerad- oder verzweigtkettigen zweiwertigen aliphatischen Rest, der wenigstens 3 Kohlenstoffatome, vorzugsweise 3 bis 10 Kohlenstoffatome umfasst, insbesondere einen Alkyl-, Alkylen-, Alkenylen- oder Alkinylenrest, der eine oder mehrere Ungesättigtheiten umfassen kann, der gegebenenfalls substituiert ist, insbesondere durch eine oder mehrere Amino-, Hydroxy-, Oxogruppen oder einen C₁-C₃-Alkylrest, wobei R₁ mit einer der Säurefunktionen einen Cyclus bilden kann, steht,
und **dadurch**, dass es die allgemeine Formel
A-X-Gln-Gly-Gly-Y (III)
aufweist, in der
A eine Verbindung der allgemeinen Formel I oder II oder der entsprechende Rest ist,
- X : Ser, Lys-Ser, Ala-Lys-Ser, Pyr-Ala-Lys-Ser, eine Bindung oder Glx-Ala-Lys-Ser ist, worin Glx: Pyro-Glu, Glu oder Gly und dessen Derivate ist,
- Y : Ser-Asn-OH
Ser-Asn-NH₂
Ser-OH
Ser-NH₂ ist,
wobei die Aminosäuren in D-, L- oder DL-Form vorliegen.

18. Peptidkonjugat nach Anspruch 17, **dadurch gekennzeichnet, dass** in der allgemeinen Formel I R stehen kann für:
- einen einfach ungesättigten Rest von cis- oder trans-Konfiguration mit der allgemeinen Formel
R₂-CH=CH-,
in welcher R₂ für einen linearen oder verzweigten Alkylrest, der wenigstens 6 Kohlenstoffatome, vorzugsweise 6 bis 16 Kohlenstoffatome umfasst und der gegebenenfalls durch eine Ami-no-, Hydroxy- oder Oxogruppe substituiert ist, steht;
- einen substituierten oder nicht-substituierten, linearen oder verzweigten aliphatischen C₁-C₂₀-Rest, insbesondere einen Alkyl-, Alkenyl- oder Alkinylrest, der eine oder mehrere Ungesättigtheiten umfassen kann und substituiert sein kann durch einen oder mehrere Reste, die ausgewählt sind aus der Gruppe, umfassend:
NH₂, OH, Oxo, Thiol oder einen cyclischen, nicht-aromatischen Rest, der 5 bis 6 Atome im Ring umfassen kann, von denen 1 oder 2 von Kohlenstoff verschieden sein können, insbesondere S, O oder N sein können, wobei die Ringe durch C₁-C₃-Alkylreste, insbesondere Methylreste substituiert sein können,
und in der allgemeinen Formel II R₁ für einen gegebenenfalls substituierten C₄-C₈-Alkylenrest steht.

19. Konjugat nach einem der Ansprüche 17 oder 18, **dadurch gekennzeichnet, dass** die Säuren der allgemeinen Formel I oder II unter Essigsäure und deren Derivaten, Pyroglutaminsäure, DL-Liponsäure und deren Derivaten, Dihydroliponsäure und Derivaten, N-Lipoyllysin, Adipinsäure, α-Aminoadipinsäure, Pimelinsäure, Sebacinsäure und deren Derivaten, den α-mono-ungesättigten Fettsäuren, für die "R₂" einen linearen oder an C7 verzweigten Alkylrest darstellt, insbesondere den Hydroxydecensäuren und Decenoylsäuren, wie trans-10-Hydroxy-Δ2-decensäure und trans-Oxo-9-decensäure, ausgewählt sind.

20. Konjugat nach einem der Ansprüche 17 bis 19, **dadurch gekennzeichnet, dass** die Aminosäuresequenz an die Säure oder das Derivat der Formel I oder II in Form von Salz, Ester oder Amid gebunden ist.

21. Konjugat nach einem der Ansprüche 17 bis 20, **dadurch gekennzeichnet, dass** es unter den folgenden Peptidderivaten ausgewählt ist:
I A - Pyr - Ala - Lys - Ser - Gln - Gly - Gly - Ser - Asn - NH₂
II A - Pyr - Ala - Lys - Ser - Gln - Gly - Gly - Ser - NH₂
III A - Ala - Lys - Ser - Gln - Gly - Gly - Ser - Asn - NH₂
IV A - Ala - Lys - Ser - Gln - Gly - Gly - Ser - NH₂
V A - Lys - Ser - Gln - Gly - Gly - Ser - Asn - NH₂
VI A - Lys - Ser - Gln - Gly - Gly - Ser - NH₂
VII A - Ser - Gln - Gly - Gly - Ser - Asn - NH₂
VIII A - Ser - Gln - Gly - Gly - Ser - NH₂
IX A - Gln - Gly - Gly - Ser - Asn - NH₂
X A - Gln - Gly - Gly - Ser - NH₂
XXIII A-Ala-Lys-Ser-Gln-Gly-Gly-Ser-Asn-OH,
wobei A eine Verbindung der Formel I oder II oder der entsprechende Rest ist, wobei die Aminosäuren in D-, L- oder DL-Form vorliegen können, wobei ein Teil der Aminosäuren in glycosylierter Form vorliegen kann.

22. Konjugat nach einem der Ansprüche 17 bis 21, **dadurch gekennzeichnet, dass** es in Form eines Metall-Peptid-Komplexes vorliegt, der chemisch oder physikalisch an ein Zinksalz gebunden ist.

23. Konjugat nach einem der Ansprüche 17 bis 22 als Arzneimittel.
